# EUROPEAN PATENT APPLICATION

(11) **EP 2 074 993 A1**
(43) Date of publication of application: **01.07.2009**
(21) Application number: 07123570.9
(22) Date of filing: 19.12.2007
(51) Int. Cl.: A61K 31/137, A61K 9/28

(54) **Venlafaxine-containing film-coated modified-release tablets**

(71) Applicant: Biovail Laboratories International S.r.l., 14018 St. Michael (BB)
(72) Inventor: Zhou, Fang, Davie, FL 33328 (US); Maes, Paul, F-69300, Caluire et Curie (FR); Frisbee, Steven, Reston, VA 20191 (US)
(74) Representative: Gates, Marie Christina Esther

(57) **Abstract**

The present invention relates to a modified release composition of at least one form of venlafaxine, which is an enhanced absorption delayed controlled release composition. The composition comprises a core comprising at least one form of venlafaxine, less than 10% of a gelling agent and a pharmaceutically acceptable excipient. The composition further comprises a modified release coating which substantially surrounds the core which provides a delayed controlled release of the at least one form of venlafaxine.

## Description

### FIELD OF THE INVENTION

The present invention relates to modified release compositions for oral administration of at least one form of venlafaxine, to processes for their preparation and to their medical use. In particular, the modified release composition relates to an enhanced absorption delayed controlled release composition of at least one form of venlafaxine.

### BACKGROUND OF THE INVENTION

An ideal dosage regimen for many medications is that by which an acceptable therapeutic concentration of drug at the site(s) of action is attained immediately and is then maintained constant for the duration of the treatment. Providing dose size and frequency of administration are correct, therapeutic "steady-state" plasma concentrations of a drug can be achieved promptly and maintained by the repetitive administration of conventional peroral dosage forms. However, there are a number of potential limitations associated with conventional peroral dosage forms. These limitations have led pharmaceutical scientists to consider presenting therapeutically active molecules in "extended-release" preparations.

Oral ingestion is the traditionally preferred route of drug administration, providing a convenient method of effectively achieving both local and systemic effects. An ideal oral drug delivery system should steadily deliver a measurable and reproducible amount of drug to the target site over a prolonged period. Extended-release (ER) delivery systems provide a uniform concentration/amount of the drug at the absorption site and thus, after absorption, allow maintenance of plasma concentrations within a therapeutic range over an extended period of time, which can minimize side effects and also reduces the frequency of administration. ER dosage forms release drug slowly, so that plasma concentrations are maintained at a therapeutic level for a prolonged period of time. Typically, these products provide numerous benefits compared with immediate-release compositions, including greater effectiveness in the treatment of chronic conditions, reduced side effects, greater convenience, and higher levels of patient compliance due to a simplified dosing schedule. Because of the above advantages, such systems form a major segment of the drug delivery market.

Many drug delivery systems have been developed with the aim of eliminating the cyclical changes in plasma drug concentration seen after the administration of a conventional delivery system. A variety of terms have been used to describe these systems: delayed release, repeat action, prolonged release, sustained release, extended release, controlled release and modified release. It is interesting to note that the USP considers that the terms controlled release, prolonged release, sustained release and extended-release are interchangeable.

Controlled-release formulations have been described in the prior art and many methods have been used to provide controlled-release pharmaceutical dosage forms in order to maintain therapeutic serum levels of medicaments and to minimize the effects of missed doses of drugs caused by a lack of patient compliance. Anti-depressants are excellent candidates for controlled-release formulations as discontinuation of these drugs, most often as a result of a lack of patient compliance due to a complicated or multiple daily dosing schedule, can often result in severe discontinuation symptoms.

Venlafaxine, chemically designated as (R/S)-1-[2-(dimethylamino)-1-(4-methoxyphenyl)ethyl]cyclohexanol or (±)-1-([α(dimethylamino)methyl]*p-*methoxybenzyl)cyclohexanol, has the following chemical structure:

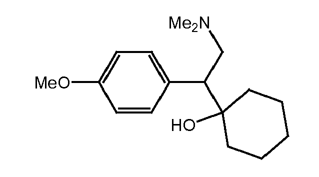

Venlafaxine is a bicyclic compound with antidepressant properties affecting chemical messengers within the brain. These chemical messengers, called neurotransmitters, can for example be serotonin, dopamine, and norepinephrine. Neurotransmitters are manufactured and released by nerve cells. The neurotransmitters travel to neighboring nerve cells and cause the cells to become more or less active. It is believed that an imbalance in these neurotransmitters is the cause of depression and also may play a role in anxiety. Venlafaxine is believed to work by inhibiting the release or affecting the action of these neurotransmitters.

Venlafaxine is chemically unrelated to other antidepressants, but is sometimes categorized as a serotonin-norepinephrine reuptake inhibitor (SNRI). At low dosages, venlafaxine blocks serotonin reuptake, similarly to a selective serotonin reuptake inhibitor (SSRI). At medium dosages, venlafaxine blocks the reuptake of norepinephrine as well as serotonin. At high dosages, venlafaxine blocks the reuptake of norepinephrine and serotonin, and is also a weak blocker of the reuptake of dopamine.

Venlafaxine is well absorbed after oral administration and its metabolism has been well documented. Following absorption, venlafaxine undergoes extensive pre-systemic metabolism in the liver, primarily to O-desmethylvenlafaxine (ODV), but also to N-desmethylvenlafaxine (NDV), N,O-didesmethylvenlafaxine (DDV), and N,N,O-tridesmethylvenlafaxine (TDV). In vitro studies indicate that the formation of ODV is catalyzed by CYP2D6; this has been confirmed in a clinical study showing that patients with low CYP2D6 levels ("poor metabolizers") had increased levels of venlafaxine and reduced levels of ODV compared to people with normal levels of CYP2D6 ("extensive metabolizers"). The differences between CYP2D6 poor and extensive metabolizers, however, are not expected to be clinically important because the sum of venlafaxine and ODV is similar in the two groups and venlafaxine and ODV are pharmacologically approximately equiactive and equipotent. Approximately 87% of a venlafaxine dose is recovered in the urine within 48 hours as unchanged venlafaxine (5%), unconjugated ODV (29%), conjugated ODV (26%), or other minor active metabolites (27%). Renal elimination of venlafaxine and its metabolites is the primary route of excretion. The metabolic pathway of venlafaxine can be summarized as follows:

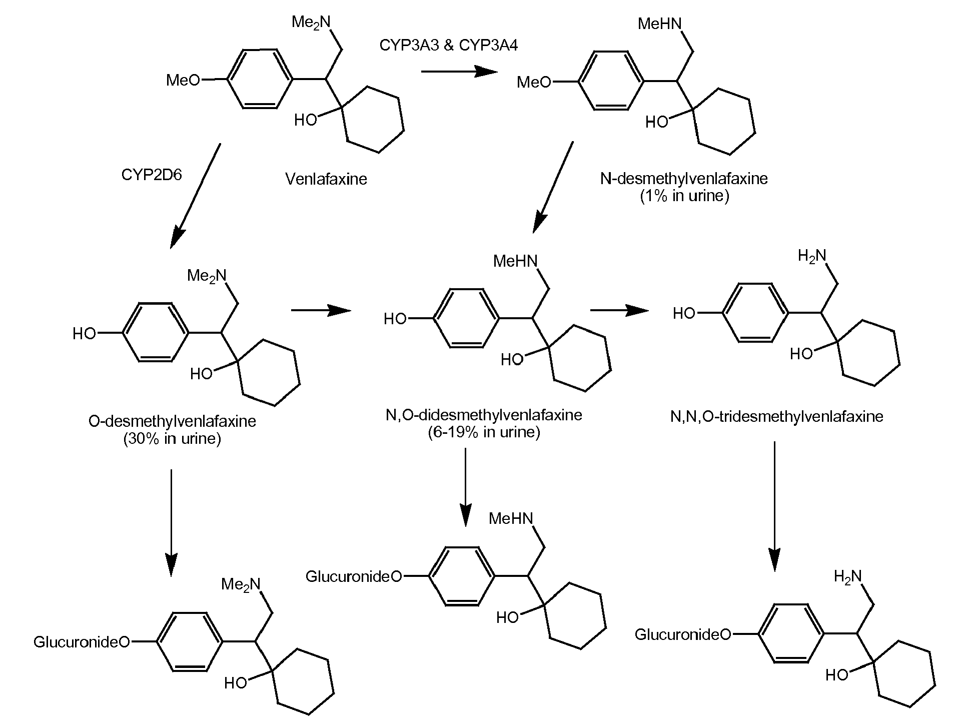

Venlafaxine's elimination half-life of about 4 hours is short, and its active metabolite has a half-life of about 8 hours. This results in venlafaxine being administered twice daily, and a lack of patient compliance in keeping to this daily dosing schedule is liable to produce discontinuation problems. Sudden discontinuation of venlafaxine can result in withdrawal symptoms, which can include, fatigue, dizziness, nausea, headache and dysphoria. Accordingly, venlafaxine is an excellent candidate for a controlled-release oral formulation.

Venlafaxine, as its hydrochloride salt, is available as a second-generation extended-release capsule and is marketed under the brand name Effexor^{®} XR for once daily use. Such a formulation has reduced the discontinuation problems seen with Effexor^{®}, the first-generation immediate-release form of venlafaxine, which is usually administered twice daily. Extended-release formulations of venlafaxine have been described in the prior art.

U.S. Pat. Nos. 6,274,171, 6,403,120, and 6,419,958, for example, disclose formulations comprising a therapeutically effective amount of venlafaxine hydrochloride in film-coated spheroids. The spheroids comprise a core having venlafaxine hydrochloride, microcrystalline cellulose, and optionally hydroxypropylmethylcellulose. The cores are coated with a mixture of ethylcellulose and hydroxypropylmethylcellulose and subsequently packaged into hard gelatin capsules. These patents also describe and claim methods and compositions for obtaining therapeutic blood plasma concentrations of venlafaxine over a twenty-four hour period with diminished incidence of nausea and emesis which comprise administering orally to a patient in need thereof, an extended-release formulation providing a peak blood plasma level of venlafaxine of no more than about 150 ng/mL 4-8 hours after administration.

U.S. Pat. No. 6,703,044 purports to teach a formulation wherein a delayed-burst release of venlafaxine is achieved at least three hours after administration resulting in dispersion of the venlafaxine mainly through the colon into the blood stream as a result of colon absorption over a period of at least 24 hours. A compressed core comprising a burst controlling agent as well as a disintegrant characterizes the formulation. The core is coated with a relatively rigid water insoluble, hydrophobic polymer, in which particles of water insoluble but hydrophilic material are embedded. These particles form channels upon contact with aqueous medium, which imbibe liquid and cause the burst-controlling agent to burst the coating thereby enabling the delayed-burst release of the venlafaxine. The '044 patent also teaches in Example 11 that the formulation surprisingly provided for a 30% higher bioavailability of the venlafaxine in fasting volunteers when compared to extended-release formulations of venlafaxine presently available on the market. The label for Effexor^{®} XR, on the other hand, states that: "Effexor XR should be administered in a single dose with food either in the morning or evening at approximately the same time each day". Example 11, the only pharmacokinetic study presented in the patent, does not show any bioavailability data in fed volunteers, and hence it is not known whether the formulation taught in the '044 patent will also provide for a higher bioavailability when administered to patients under the conditions recommended by the Effexor^{®} XR label, i.e. under fed conditions. The '044 patent does not provide any data on the adverse events or side effect profile of the claimed composition.

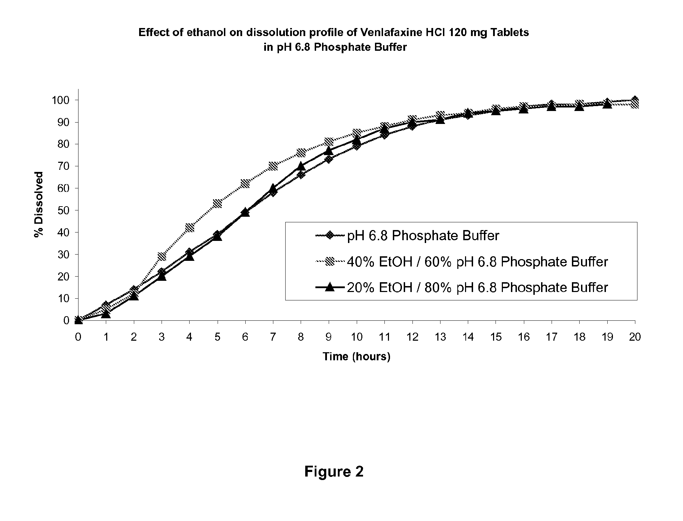

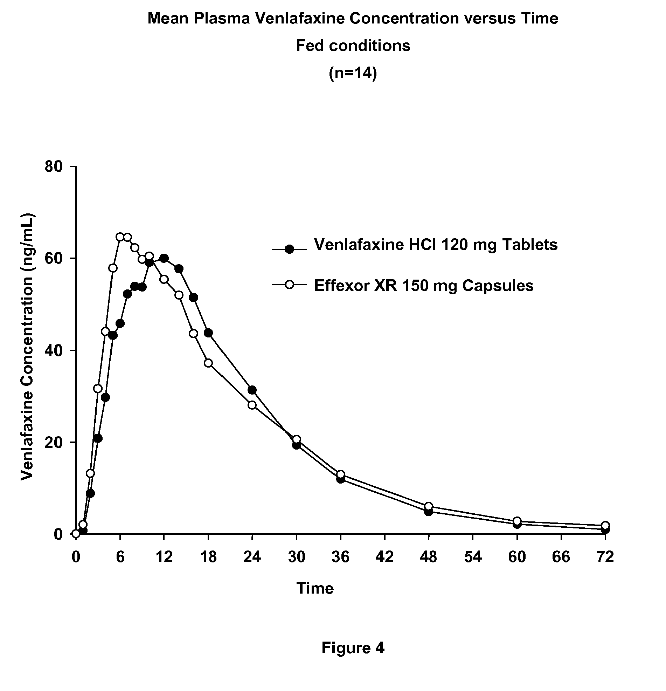

The disclosures of the '120, '171, and '958 patents discussed above teach that "... various attempts to produce extended release tablets of venlafaxine hydrochloride by hydrogel technology proved to be fruitless because the compressed tablets were either physically unstable (poor compressibility or capping problems) or dissolved too rapidly in dissolution studies" (col. 4, lines 60-64 of the '120, 171, and '958 patents). Makhija and Vavia of the Pharmaceutical Division, Dept. of Chemical Technology (Autonomous), University of Mumbai, India, however, describe a once daily sustained-release tablet of venlafaxine using hydrogel technology (Eur. J. Pharmaceut. Biopharmaceut. 2002. 54:9-15). The Makhija and Vavia reference teaches a once daily sustained-release tablet of venlafaxine hydrochloride using an uncoated matrix system based on swellable as well as non-swellable polymers. Interestingly, the bioavailability of venlafaxine for this formulation, like that of the '044 formulation is, also significantly improved over that of Effexor^{®} XR even though there does not appear to be any delay in the release of the drug in vitro (FIG. 2) or in vivo (FIG. 4). However, like the'044 invention, the formulation was administered to individuals in the fasted state. Accordingly, it is not known whether the Makhija and Vavia formulation would provide a higher bioavailability in the fed state. Finally, the Makhija and Vavia reference does not teach the effect of their formulation on the incidence and frequency of any adverse events in comparison to Effexor^{®} XR.

Delayed release formulations comprising venlafaxine as the active agent have also been described in the prior art. For example, U.S. patent application Ser. No. 10/244,059, published as US 2003/0091634A1 on May 15, 2003 and U.S. patent application Ser. No. 09/953,101, published as US 2003/0059466A1 on Mar. 27, 2003 both describe a delayed release tablet, comprising a core comprising 10 to 70% of active agent, 10 to 80% of a gelling agent, and optional conventional excipients; and a coating consisting essentially by weight, based on the coating weight, of 20 to 85% of a water-insoluble, water-permeable film-forming polymer, of 10 to 75% of a water-soluble polymer or substance and 3 to 40% of a plasticizer.

Venlafaxine is currently among the top five prescribed antidepressant medications within the SSRI/SNRI category of antidepressants. However, only one once-a-day oral dosage form comprising venlafaxine hydrochloride is currently being marketed, under the trade name Effexor^{®} XR. Given the efficacy of venlafaxine, a once-a-day oral composition comprising at least one form of venlafaxine capable of providing a higher bioavailability compared to the currently marketed Effexor^{®} XR 150 mg capsules, with a reduced or similar side effect or adverse event profile would be desirable. Such a composition can also allow for a composition having an absolute amount of the active drug that is less than the amount in the reference product, thereby providing for a better safety profile. Furthermore, a composition comprising at least one form of venlafaxine which shows a reduced ethanol-induced dose dumping effect, such that there is little or no increase in the release of venlafaxine from the composition in the presence of ethanol compared to the release of venlafaxine from the dosage form in the absence of ethanol, would also be desirable.

### SUMMARY OF THE INVENTION

The present invention relates to a modified release composition of at least one form of venlafaxine.

In one embodiment of the invention, the modified release composition of the at least one form of venlafaxine is a delayed controlled release pharmaceutical composition for oral administration suitable for once daily dosing comprising: a) a core comprising by weight of the core dry weight from about 10% to about 90% of at least one form of venlafaxine, less than 10% of a gelling agent, and optional conventional excipients, and b) a modified release coat substantially surrounding said core; wherein said composition provides a delayed controlled release of said at least one form of venlafaxine such that no more that 25% of the at least one form of venlafaxine is released after about 2 hours, about 15% to about 45% of the at least one form of venlafaxine is released after about 4 hours, about 50% to about 90% of the at least one form of venlafaxine is released after about 8 hours, no less than about 70% of the at least one form of venlafaxine is released after about 12 hours and no less than about 80% of the at least one form of venlafaxine is released after about 16 hours when tested using USP Apparatus 1 in 1000 mL of pH 6.8 phosphate buffer at 75 rpm at 37°C ±.0.5°C.

In another embodiment of the invention, the modified release composition of the at least one form of venlafaxine is a delayed controlled release pharmaceutical composition for oral administration suitable for once daily dosing comprising: a) a core comprising by weight of the core dry weight from about 10% to about 90% of at least one form of venlafaxine, less than 10% of a gelling agent, and optional conventional excipients; and b) a modified release coat substantially surrounding said core, said coat comprising by weight of the coat dry weight from about 20% to about 85% of a water-insoluble water-permeable film-forming polymer, from about 10% to about 75% of a water-soluble polymer or substance and from about 3% to about 40% of a plasticizer; wherein said composition provides a delayed controlled release of said at least one form of venlafaxine such that no more that 25% of the at least one form of venlafaxine is released after about 2 hours, about 15% to about 45% of the at least one form of venlafaxine is released after about 4 hours, about 50% to about 90% of the at least one form of venlafaxine is released after about 8 hours, no less than about 70% of the at least one form of venlafaxine is released after about 12 hours and no less than about 80% of the at least one form of venlafaxine is released after about 16 hours when tested using USP Apparatus 1 in 1000 mL of pH 6.8 phosphate buffer at 75 rpm at 37°C ±.0.5°C.

In another embodiment of the invention, the modified release composition of the at least one form of venlafaxine is an enhanced absorption delayed controlled release pharmaceutical composition for oral administration suitable for once daily dosing comprising:
a) a core comprising by weight of the core dry weight from about 10% to about 90% of at least one form of venlafaxine, less than 10% of a gelling agent, and optional conventional excipients;
   wherein the gelling agent comprises hydroxypropylmethylcellulose and polyvinyl alcohol; and
b) a modified release coat substantially surrounding said core, said coat comprising at least one water-insoluble, water-permeable, film-forming polymer, at least one water-soluble polymer or substance and at least one plasticizer;
wherein said composition provides a delayed controlled release of said at least one form of venlafaxine such that no more that 25% of the at least one form of venlafaxine is released after about 2 hours, about 15% to about 45% of the at least one form of venlafaxine is released after about 4 hours, about 50% to about 90% of the at least one form of venlafaxine is released after about 8 hours, no less than about 70% of the at least one form of venlafaxine is released after about 12 hours and no less than about 80% of the at least one form of venlafaxine is released after about 16 hours when tested using USP Apparatus 1 in 1000 ml of pH 6.8 phosphate buffer at 75 rpm at 37°C ± 0.5°C.

In another embodiment of the invention, the modified release composition of the at least one form of venlafaxine is an enhanced absorption delayed controlled release pharmaceutical composition for oral administration suitable for once daily dosing comprising:
a) a core comprising by weight of the core dry weight from about 10% to about 90% of at least one form of venlafaxine, less than 10% of a gelling agent, and optional conventional excipients; and
b) a modified release coat substantially surrounding the core, the coat comprising at least one water-insoluble, water-permeable, film-forming polymer, at least one water-soluble polymer or substance and at least one plasticizer;
wherein the composition provides a delayed controlled release of the at least one form of venlafaxine such that no more that 25% of the at least one form of venlafaxine is released after about 2 hours, about 15% to about 45% of the at least one form of venlafaxine is released after about 4 hours, about 50% to about 90% of the at least one form of venlafaxine is released after about 8 hours, no less than about 70% of the at least one form of venlafaxine is released after about 12 hours and no less than about 80% of the at least one form of venlafaxine is released after about 16 hours when tested using USP Apparatus 1 in 1000 ml of pH 6.8 phosphate buffer at 75 rpm at 37°C ± 0.5°C; and
wherein the composition shows a reduced ethanol-induced dose dumping effect compared to a reference composition, wherein the reference composition is a commercially available extended release formulation of venlafaxine.

In another embodiment of the invention, the modified release composition of the at least one form of venlafaxine is an enhanced absorption delayed controlled release pharmaceutical composition for oral administration suitable for once daily dosing comprising:
a) a core comprising weight of the core dry weight from about 10% to about 90% of at least one form of venlafaxine, less than 10% of a gelling agent, and optional conventional excipients;
   wherein the gelling agent comprises hydroxypropylmethylcellulose and polyvinyl alcohol; and
b) a modified release coat substantially surrounding the core, the coating comprising by weight of the coat dry weight from about 20% to about 85% of a water-insoluble water-permeable film-forming polymer, from about 10% to about 75% of a water-soluble polymer or substance and from about 3% to about 40% of a plasticizer;
wherein the composition provides a delayed controlled release of the at least one form of venlafaxine such that no more that 25% of the at least one form of venlafaxine is released after about 2 hours, about 15% to about 45% of the at least one form of venlafaxine is released after about 4 hours, about 50% to about 90% of the at least one form of venlafaxine is released after about 8 hours, no less than about 70% of the at least one form of venlafaxine is released after about 12 hours and no less than about 80% of the at least one form of Venlafaxine is released after about 16 hours when tested using USP Apparatus 1 in 1000 ml of pH 6.8 phosphate buffer at 75 rpm at 37°C ± 0.5°C.

### BRIEF DESCRIPTION OF THE DRAWINGS

Figure 1 is a graph showing the dissolution profile in phosphate buffer, pH 6.8 of uncoated venlafaxine 120 mg tablet cores prepared according to Example 5.
Figure 2 is a graph showing the effect of ethanol, at concentrations of 20% and 40%, on the dissolution profile in phosphate buffer, pH 6.8 of venlafaxine 120 mg tablets prepared according to Example 5.
Figure 3 is a graph showing the effect of ethanol, at a concentration of 40%, on the dissolution profile in deionized water of Effexor^{®} XR 150 mg capsules.
Figure 4 is a graph comparing the mean plasma venlafaxine concentrations under fed conditions for venlafaxine 120 mg tablets prepared according to Example 5 and for Effexor^{®} XR 150 mg capsules.
Figure 5 is a graph comparing the mean plasma O-desmethylvenlafaxine concentrations under fed conditions for venlafaxine 120 mg tablets prepared according to Example 5 and for Effexor^{®} XR 150 mg capsules.
Figure 6 is a graph comparing the mean plasma Pharmacologic Activity-Weighted Composite (PAWC) concentrations under fed conditions for venlafaxine 120 mg tablets prepared according to Example 5 and for Effexor^{®} XR 150 mg capsules.
Figure 7 is a graph comparing the mean plasma venlafaxine concentrations under fasted conditions for venlafaxine 120 mg tablets prepared according to Example 5 and for Effexor^{®} XR 150 mg capsules.
Figure 8 is a graph comparing the mean plasma O-desmethylvenlafaxine concentrations under fasted conditions for venlafaxine 120 mg tablets prepared according to Example 5 and for Effexor^{®} XR 150 mg capsules.
Figure 9 is a graph comparing the mean plasma Pharmacologic Activity-Weighted Composite (PAWC) concentrations under fasted conditions for venlafaxine 120 mg tablets prepared according to Example 5 and for Effexor^{®} XR 150 mg capsules.

### DETAILED DESCRIPTION OF THE INVENTION

The present invention is directed to a modified release pharmaceutical composition of venlafaxine. In particular, the composition is an enhanced absorption delayed controlled release composition of the at least one form of venlafaxine comprising a core and a modified release coating, which substantially surrounds the core, wherein the composition provides a delayed controlled release of the at least one form of venlafaxine. The enhanced absorption delayed controlled release oral dosage form of the invention has a higher bioavailability and is expected to have reduced or similar side effects or adverse events when compared to the reference product.

As used interchangeably herein, the terms "enhanced absorption composition" or "enhanced absorption dosage form" are defined to mean a composition which exhibits increased bioavailability when compared to the bioavailability of a reference composition. The reference composition can be, for example, a commercially available formulation of the same active ingredient, including but not limited to a commercially available modified release or extended release formulation of the same active ingredient. In the case of at least one form of venlafaxine, suitable reference compositions include, but are not limited to, the commercial formulation known as Effexor^{®} XR. The increased bioavailability may be apparent as an increase in the values of pharmacokinetic parameters indicative of bioavailability, including but not limited to Cₘₐₓ and the area under the concentration-time curve (AUC). For example, when administered at the same dosage strength, pharmacokinetic parameters, including but not limited to Cₘₐₓ and AUC, of an enhanced absorption test composition will be higher than those of the corresponding reference composition. Alternatively, an enhanced absorption test composition will show bioequivalence to the corresponding reference composition when the enhanced absorption test composition is administered at a lower dose than the corresponding reference composition.

In at least one embodiment, the enhanced absorption delayed controlled release composition of the present invention shows bioequivalence to a reference composition when the reference composition is administered at a dose which is at least about 1.20 times the dose at which the enhanced absorption delayed controlled release composition is administered. As used herein, the term "bioequivalence" is defined to mean that the 90% confidence intervals of the ratios of the geometric means of the AUC and Cₘₐₓ values of the at least one form of venlafaxine for the test composition to the geometric means of the AUC and Cₘₐₓ values of the at least one form of venlafaxine for the reference composition fall between 80.00% and 125.00%.

In at least one embodiment, the combined geometric mean ratio of the AUC or Cₘₐₓ values of the enhanced absorption delayed controlled release composition of the invention to the AUC or Cₘₐₓ values of the reference composition for the at least one form of venlafaxine is greater than 1 when the enhanced absorption delayed controlled release composition and the reference composition are administered at the same dosage strength. As used herein, the "geometric mean ratio" is defined to mean the geometric mean of the enhanced absorption delayed controlled release composition of the invention divided by the geometric mean of the reference composition for a particular pharmacokinetic parameter. Thus, the "geometric mean ratio" for the AUC₀₋ₜ for venlafaxine, for example, means the geometric mean of the AUC₀₋ₜ for venlafaxine of the enhanced absorption delayed controlled release composition of the invention divided by the geometric mean of the AUC₀₋ₜ for venlafaxine of the reference composition. Thus, if the geometric mean for the AUC₀₋ₜ for venlafaxine of the enhanced absorption delayed controlled release composition of the invention is X and the geometric mean for the AUC₀₋ₜ for venlafaxine for the reference composition is *Y*, then the geometric mean ratio for the AUC₀₋ₜ for venlafaxine is *XIY.* Similarly, if the geometric mean for the AUC₀₋ₜ for O-desmethylvenlafaxine of the enhanced absorption delayed controlled release composition of the invention is A and the geometric mean for the AUC₀₋ₜ for O-desmethylvenlafaxine of the reference composition is *B*, then the geometric mean ratio for the AUC₀₋ₜ for O-desmethylvenlafaxine is *A*/*B.* As used herein, the "combined geometric mean ratio" means the geometric mean ratio of venlafaxine for a particular pharmacokinetic parameter plus the geometric mean ratio of O-desmethylvenlafaxine for the same pharmacokinetic parameter. To use the above example, the combined geometric mean ratio for the AUC₀₋ₜ is therefore [(*X*/*Y*) + (*A*/*B*)].

### The Tablet Cores

As used herein, the terms "core" or "tablet core", used interchangeably, are defined to mean an uncoated tablet comprising at least one form of venlafaxine, a gelling agent and optionally conventional excipients. The tablet cores are subsequently coated so as to be substantially completely surrounded by a modified release polymer coat, such that the composition provides a delayed controlled release of the at least one form of venlafaxine.

As used herein, the term "at least one form of venlafaxine" is defined to mean venlafaxine; stereoisomers thereof, including but not limited to individual enantiomers and mixtures of enantiomers, including but not limited to racemic mixtures; active metabolites thereof; stereoisomers of active metabolites thereof, including but not limited to individual enantiomers and mixtures of enantiomers including but not limited to racemic mixtures; pharmaceutically acceptable salts thereof, including but not limited to pharmaceutically acceptable salts of venlafaxine, active metabolites thereof, and enantiomers and mixtures of enantiomers of venlafaxine and active metabolites thereof; and combinations thereof. Active metabolites of venlafaxine include but are not limited to O-desmethylvenlafaxine, N-desmethylvenlafaxine, N,O-didesmethylvenlafaxine, N,N,O-tridesmethylvenlafaxine and active conjugates and other metabolites thereof.

In at least one embodiment, the at least one form of venlafaxine is present in the core from about 10 to about 90% by weight of the core dry weight. In at least one embodiment, the at least one form of venlafaxine is present in the core from about 20 to about 60% by weight of the core dry weight. In at least one embodiment, the at least one form of venlafaxine is present in the core from about 35% to about 45% by weight of the core dry weight. In at least one embodiment, the at least one form of venlafaxine is present in the core at about 42% by weight of the core dry weight. In at least one embodiment, the composition comprises a pharmaceutically effective amount of the at least one form of venlafaxine of from about 0.5 to about 1000 mg. In at least one embodiment, the composition comprises a pharmaceutically effective amount of the at least one form of venlafaxine of from about 20 to about 200 mg. In at least one embodiment, the composition comprises a pharmaceutically effective amount of the at least one form of venlafaxine of from about 100 to about 200 mg. In at least one embodiment, the composition comprises a pharmaceutically effective amount of the at least one form of venlafaxine of about 30 mg, about 60 mg, about 120 mg or about 180 mg.

The term "effective amount" as used herein means that a "pharmaceutically effective amount" is contemplated. A "pharmaceutically effective amount" is the amount or quantity of the at least one form of venlafaxine in a dosage form of the invention sufficient to elicit an appreciable clinical or therapeutic response when administered, in single or multiple doses to a patient in need thereof. It will be appreciated that the precise therapeutic dose will depend on the age and condition of the patient and the nature of the condition to be treated and will be at the ultimate discretion of the attendant physician. It is well known to the skilled artisan that the therapeutically or clinically effective amount for a certain indication can be determined by conducting clinical studies using dosage forms that contain a pharmaceutically effective amount of the at least one form of venlafaxine.

The term "pharmaceutically acceptable salt" as used herein is intended to mean a salt of the at least one form of venlafaxine which is, within the scope of sound medical judgment, suitable for use in contact with the tissues of humans and lower animals without undue toxicity, irritation, allergic response, and the like, commensurate with a reasonable benefit/risk ratio, generally water or oil-soluble or dispersible, and effective for their intended use. The term includes pharmaceutically acceptable acid addition salts. Lists of suitable salts are found in, for example, S. M. Berge et al., J. Pharm. Sci. (1977) 66(1):1-19.

The term "pharmaceutically-acceptable acid addition salt" as used herein is intended to mean those salts which retain the biological effectiveness and properties of the free bases and which are not biologically or otherwise undesirable, formed with inorganic acids including but not limited to hydrochloric acid, hydrobromic acid, hydroiodic acid, sulfuric acid, sulfurous acid, sulfamic acid, nitric acid, phosphoric acid, carbonic acid and the like, and organic acids including but not limited to acetic acid, acrylic acid, trifluoroacetic acid, adipic acid, ascorbic acid, aspartic acid, benzenesulfonic acid, benzoic acid, p-bromobenzenesulfonic acid, butynedioic acid, butyric acid, camphoric acid, camphorsulfonic acid, caproic acid, caprylic acid, chlorobenzoic acid, cinnamic acid, citric acid, decanoic acid, digluconic acid, dinitrobenzoic acid, ethanesulfonic acid, formic acid, fumaric acid, gluconic acid, glutamic acid, glycolic acid, glycerophosphoric acid, hemisulfic acid, hexanoic acid, hexynedioic acid, heptanoic acid, hydroxybenzoic acid, gamma-hydroxybutyric acid, 2-hydroxyethanesulfonic acid (isethionic acid), hydroxymaleic acid, isobutyric acid, lactic acid, maleic acid, malic acid, malonic acid, mandelic acid, mesitylenesulfonic acid, methanesulfonic acid, methoxybenzoic acid, methylbenzoic acid, mucic acid, naphthalenesulfonic acid, nicotinic acid, 2-naphthalenesulfonic acid, oxalic acid, pamoic acid, pantothenic acid, pectinic acid, phenylacetic acid, phenylbutyric acid, 3-phenylpropionic acid, phthalic acid, pivalic acid, propanesulfonic acid, propiolic acid, propionic acid, pyruvic acid, salicylic acid, sebacic acid, stearic acid, suberic acid, succinic acid, sulfanilic acid, tartaric acid, p-toluenesulfonic acid, undecanoic acid, xylenesulfonic acid, and the like. The hydrochloric salt is the most preferred. Other salts, such as venlafaxine maleate and venlafaxine besylate have been described in International patent application Nos. PCT/EP03/03319 (WO 03/082805) and PCT/EP03/03318 (WO 03/082804) respectively.

Venlafaxine, or the venlafaxine in the pharmaceutically acceptable salts of venlafaxine, can be any form of venlafaxine. For example, venlafaxine has one optically active carbon, thus allowing for existence of two enantiomers and a racemate. Both enantiomers are pharmaceutically active. Thus, the effective amount of the preferred active in the core of the oral dosage form of the invention, venlafaxine hydrochloride, can be based on the racemate or mixture of enantiomers of venlafaxine or on the pure or substantially pure (+) or (-) enantiomer of venlafaxine. The (+) and (-) enantiomers of venlafaxine have been described in U.S. Pat. Nos. 6,197,828 and 6,342,533 respectively. All such forms of venlafaxine are included within the meaning of the term "venlafaxine", "pharmaceutically acceptable salts of venlafaxine", "active metabolite of venlafaxine", and "pharmaceutically acceptable salts of an active metabolite of venlafaxine".

The at least one gelling agent comprises a substance that is hydrophilic in nature and which is capable of behaving like a hydrophilic matrix. Examples of gelling agents are described in U.S. patent Ser. No. 10/244,059, published May 15, 2003 as US 2003/0091634 and in the Handbook of Pharmaceutical Excipients, 4th Edition (2003), edited by Rowe et al. and published by the Pharmaceutical Press and the American Pharmaceutical Association and include but are not limited to cellulose polymers and their derivatives, polysaccharides and their derivatives, polyalkylene oxides, polyethylene glycols, chitosan, poly(vinyl alcohol), xanthan gum, maleic anhydride copolymers, poly(vinyl pyrrolidone), starch and starch-based polymers, poly (2-ethyl-2-oxazoline), poly(ethyleneimine), polyurethane hydrogels, crosslinked polyacrylic acids and their derivatives, and mixtures thereof. Cellulose polymers and their derivatives contemplated for use as gelling agents include but are not limited to microcrystalline cellulose and alkylsubstituted cellulosic polymers such as, for example, methylcellulose, hydroxymethylcellulose, hydroxyethylcellulose (NATRASOL^{®} 250HX NF), hydroxypropylcellulose, hydroxypropylmethylcellulose, carboxymethylcellulose, and mixtures thereof. Polyalkylene oxides contemplated for use as gelling agents include but are not limited to poly(ethylene oxide), which term is used herein to denote a linear polymer of unsubstituted ethylene oxide. Polysaccharides and their derivatives, both natural and modified (semi-synthetic), contemplated for use as gelling agents include, but are not limited to, dextran, xanthan gum, gellan gum, welan gum, rhamsan gum, and mixtures thereof. Crosslinked polyacrylic acids that will desirably be used as gelling agents include but are not limited to CARBOPOL^{®} NF grades 971 P, 974P and 934P and polymers known as WATER LOCK^{®}, which are starch/acrylates/acrylamide copolymers.

The at least one gelling agent is present in an amount less than 10%, for example about 9.5%, about 9%, about 8.5%, about 8%, about 7.5%, about 7%, about 6.5%, about 6%, about 5.5%, about 5%, about 4.5%, about 4%, about 3.5%, about 3%, about 2.5%, about 2%, about 1.5%, about 1 % or about 0.5% by weight of the core dry weight, including all values and subranges therebetween. In at least one embodiment, the gelling agent is polyvinyl alcohol present at about 1.5% (for a 180, 120, or 60 mg tablet of venlafaxine) or about 1 % (for a 30 mg tablet of venlafaxine) by weight of the core dry weight. In at least one embodiment, the gelling agent is polyvinyl alcohol present at about 3.5% by weight of the core dry weight. In at least one embodiment, the at least one gelling agent can comprise a mixture of two or more gelling agents as long as the total amount of the gelling agent is less than 10% of the core dry weight. For example, the mixture of gelling agents can comprise a mixture of polyvinyl alcohol and methylcellulose or a mixture of polyvinyl alcohol and hydroxypropylmethylcellulose. Examples of commercially available hydroxypropylmethylcelluloses include but are not limited to METHOCEL^{®} E (USP type 2910), METHOCEL^{®} F (USP type 2906), METHOCEL^{®} J (USP type 1828), METHOCEL^{®} K (USP type 2201), and METHOCEL^{®} 310 Series (Dow Chemical Company). In these combinations, the polyvinyl alcohol can comprise from about 1% to about 4% by weight of the core dry weight and the methylcellulose or hydroxypropylmethylcellulose can comprise from about 4% to about 6% by weight of the core dry weight. In at least one embodiment, the combination of gelling agents can comprise a mixture of polyvinyl alcohol at about 1.5% and methylcellulose or hydroxypropylmethylcellulose at about 5% by weight of the core dry weight. In at least one embodiment, the combination of gelling agents can comprise a mixture of polyvinyl alcohol in an amount of from about 3% to about 3.5% and methylcellulose or hydroxypropylmethylcellulose at about 5% by weight of the core dry weight. In at least one embodiment, the combination of gelling agents can comprise a mixture of polyvinyl alcohol at about 1.5% and hydroxypropylmethylcellulose at about 5% by weight of the core dry weight. In at least one embodiment, the combination of gelling agents can comprise a mixture of polyvinyl alcohol at about 3.5% of the core dry weight and hydroxypropylmethylcellulose at about 5% of the core dry weight.

Without wishing to be bound to any particular theory it is believed that a low gelling formulation i.e., a formulation having less than 10% of a gelling agent, in addition to the enhanced absorption delayed controlled release characteristic of the composition described herein, can help to alleviate a problem observed with formulations having higher amounts of gelling agent; namely that such high gelling agent formulations can be more difficult to manufacture on a larger, commercial scale. Reduction of the amount of gelling agent in the core to less than 10% can provide a composition which can be granulated by a fluid bed granulation process, a process which is more reproducibly scaled up to manufacture on a commercial scale.

In addition to the above ingredients, a series of excipients can be included in the tablet to ensure that the tableting operation can run satisfactorily and to ensure that tablets of specified quality are prepared. Depending on the intended main function, excipients to be used in tablets are subcategorized into different groups. However, one excipient can affect the properties of a tablet in a series of ways, and many excipients used in tablet compositions can thus be described as being multifunctional.

For example, the core can further comprise at least one lubricant. Lubricants are added to pharmaceutical formulations to ensure that tablet formation and ejection can occur with low friction between the solid and the die wall. High friction during tabletting can cause a series of problems, including inadequate tablet quality (capping or even fragmentation of tablets during ejection, and vertical scratches on tablet edges) and can even stop production. Non-limiting examples of lubricants useful for the oral dosage form described herein include magnesium stearate, talc, sodium stearyl fumarate, calcium stearate, silica gel, colloidal silicon dioxide, Compritol 888 ATO, glyceryl behenate, stearic acid, hydrogenated vegetable oils (such as hydrogenated cottonseed oil (Sterotex^{®}), hydrogenated soybean oil (Sterotex^{®} HM) and hydrogenated soybean oil & castor wax (Sterotex^{®} K), stearyl alcohol, leucine, polyethylene glycol (MW 4000 and higher), and mixtures thereof. The at least one lubricant can be present in an amount from about 0.02% to about 5% by weight of the core dry weight. In at least one embodiment, the lubricant is glyceryl behenate and is present at about 3% by weight of the core dry weight. In at least one embodiment, the lubricant is magnesium stearate and is present at about 0.5% by weight of the core dry weight.

Some oral dosage forms require the incorporation of one or more excipients into the dosage form to increase the bulk volume of the powder and hence the size of the dosage form. Accordingly, the core can further comprise at least one filler (or diluent). Non-limiting examples of the at least one filler useful for the oral dosage form described herein include lactose monohydrate, anhydrous lactose, mannitol, sorbitol, microcrystalline cellulose, dibasic calcium, and calcium sulfate. Mixtures of fillers can also be used. The at least one filler is preferably present up to about 75% by weight of the core dry weight. In at least one embodiment, the at least one filler is present at from about 40% to about 75% by weight of the core dry weight. The preferred filler is lactose monohydrate. Most preferably, the lactose monohydrate is of the type called Lactose #315 Spray Dried, which is a mixture of a specially prepared pure a-lactose monohydrate along with a small amount of amorphous lactose. In at least one embodiment, the Lactose #315 Spray Dried is present at about 53% (for a 180, 120, or 60 mg tablet of venlafaxine) or 72% (for a 30 mg tablet of venlafaxine) by weight of the core dry weight. In at least one embodiment, the Lactose #315 Spray Dried is present at about 40% to about 50% by weight of the core dry weight. The at least one form of venlafaxine and filler, preferably Lactose 315 (Spray Dried) are first granulated with an aqueous solution of the gelling agent, preferably polyvinyl alcohol, in a suitable fluid bed granulator apparatus. The at least one form of venlafaxine and filler are optionally mixed with a gelling agent, such as, for example, hydroxypropylmethylcellulose, prior to granulation with the aqueous solution of the gelling agent. The granulate is subsequently dried and sieved through a suitable screen, such as, for example, a 1.4 mm screen or a 12 mesh screen. The sized granules are next blended with more filler in a V-blender or any other suitable blending apparatus together with a lubricant, preferably glyceryl behenate or magnesium stearate, and if necessary, any other additional inert excipients, which can improve processing of the oral dosage form of the invention. Alternatively, the ingredients can also be dry blended and directly compressed by methods known in the art.

The dried milled granules are then pressed into tablets and are hereinafter referred to as "tablet cores" or simply as "cores". Tablet cores can be obtained by the use of standard techniques and equipment well known to the skilled artisan. Preferably, the tablet cores are obtained by a rotary press (also referred to as a multi-station press) fitted with suitable punches. At this stage, the core formulation is an immediate-release formulation resulting in at least about 90% release of the at least one form of venlafaxine in about 30 minutes.

### The Coat

The cores are next coated with a polymer coat designed to achieve a delayed controlled-release of the at least one form of venlafaxine. The coat is designed to achieve an in vitro release profile of the at least one form of venlafaxine, preferably the hydrochloride salt of venlafaxine, such that the composition, when tested in vitro using the USP type I method at 75 rpm in 1000 mL phosphate buffer pH 6.8 at 37°C releases no more that 25% of the at least one form of venlafaxine after about 2 hours, about 15% to about 45% of the at least one form of venlafaxine after about 4 hours, about 50% to about 90% of the at least one form of venlafaxine after about 8 hours, no less than about 70% of the at least one form of venlafaxine is after about 12 hours and no less than about 80% of the at least one form of venlafaxine after about 16 hours. The preferred polymer coat for achieving the delayed controlled-release of the at least one form of venlafaxine is a semi-permeable coat which is permeable to venlafaxine and does not have a preformed pore as described for example in U.S. Pat. No. 5,654,005. The semi-permeable coat comprises at least one water-insoluble, water-permeable film-forming polymer, at least one water-soluble polymer or substance, and at least one plasticizer. The polymer coat is designed such that the integrity of the coat remains intact and the coat does not dissolve and/or disintegrate for a period of at least about 24 hours in purified water, 0.1 N HCl, Simulated Gastric Fluid (SGF) pH 1.2, or pH 6.8 phosphate buffer. As these conditions are intended to mimic the in vivo condition, it is believed that the integrity of the polymer coat will also remain intact and that the coat will not dissolve and/or disintegrate in the gastrointestinal tract. The polymer coat described herein is thus fundamentally different from the polymer coat described in U.S. Pat. No. 6,117,453, which is a quick-dissolving film, and U.S. Pat. No. 6,703,044, which is a rigid film designed to burst, thereby releasing the active from the core.

Non-limiting examples of the at least one water-insoluble, water permeable film-forming polymer include but are not limited to a cellulose ether, such as ethylcellulose, a cellulose ester, such as cellulose acetate, methacrylic acid derivatives, aqueous ethylcellulose dispersions such as Surelease^{®}, aqueous enteric coating systems such as Sureteric^{®}, and aqueous acrylic enteric systems such as Acryl-EZE^{®}. Combinations are also permitted. In at least one embodiment, the at least one water-insoluble, water-permeable film forming polymer is present in an amount ranging from about 20 to about 85%. In at least one embodiment, the at least one water-insoluble, water-permeable film forming polymer is present in an amount ranging from about 53 to about 62% by weight of the coating dry weight. In at least one embodiment, the at least one water-insoluble, water-permeable film forming polymer is present in an amount of about 60% by weight of the coating dry weight. In at least one embodiment, the at least one water-insoluble, water-permeable film forming polymer is present in an amount ranging from about 54% to about 56% by weight of the coating dry weight. In at least one embodiment, ethylcellulose is the at least one water-insoluble, water-permeable film-forming polymer and is present from about 53 to about 62% by weight of the coating dry weight. In at least one embodiment, the ethylcellulose is present at about 60% of the coating dry weight. In at least one embodiment, the ethylcellulose is present in an amount ranging from about 54% to about 56% of the coating dry weight.

The at least one water-soluble polymer or substance can be a partially or totally water-soluble hydrophilic substance intended to modulate the film permeability to the outside aqueous medium. Non-limiting examples of the at least one water-soluble polymer or substance include but are not limited to polyvinylpyrrolidone, polyethyleneglycol, hydroxypropylmethylcellulose, hydrated colloidal silica, sucrose, mannitol, and combinations thereof. In at least one embodiment, the at least one water-soluble polymer comprises from about 10 to about 75% by weight of the coating dry weight. In at least one embodiment, the at least one water-soluble polymer comprises from about 20% to about 30% by weight of the coating dry weight. In at least one embodiment, the at least one water-soluble polymer comprises about 23% to about 26% by weight of the coating dry weight. In at least one embodiment, the at least one water-soluble polymer comprises about 25% to about 35% by weight of the coating dry weight. In at least one embodiment, the at least one water-soluble polymer comprises about 30% to about 33% by weight of the coating dry weight. In at least one embodiment, the at least one water-soluble polymer is polyvinylpyrrolidone and comprises from about 23% to about 26% by weight of the coating dry weight. In at least one embodiment, the at least one water-soluble polymer is polyvinylpyrrolidone and comprises from about 30% to about 33% by weight of the coating dry weight.

Plasticizers are generally added to film coating formulations to modify the physical properties of the polymer to make it more usable. The amount and choice of the plasticizer may affect the dissolution characteristics of a tablet, as well as its physical and chemical stability. One important property of plasticizers is their ability to make a coat more elastic and pliable, thereby decreasing the coat's brittleness. Non-limiting examples of the at least one plasticizer useful for the preferred polymer coat include but are not limited to polyols, such as polyethylene glycol of various molecular weights, organic esters, such as diethyl phthalate or triethyl citrate, dibutyl sebacate, dibutyl pthalate, and oils/glycerides such as fractionated coconut oil or castor oil. Combinations are permitted. In at least one embodiment, the at least one plasticizer is present from about 3 to about 40% by weight of the coating dry weight. In at least one embodiment, the at least one plasticizer is present from about 13 to about 18% by weight of the coating dry weight. In at least one embodiment, the at least one plasticizer is present from about 15% to about 17% by weight of the coating dry weight. In at least one embodiment, the at least one plasticizer is present from about 10% to about 17% by weight of the coating dry weight. In at least one embodiment, the at least one plasticizer is present from about 12% to about 14% by weight of the coating dry weight. In at least one embodiment, the at least one plasticizer is dibutyl sebacate, and is present in an amount from about 15% to about 17% by weight of the coating dry weight. In at least one embodiment, the at least one plasticizer is dibutyl sebacate, and is present in an amount from about 10% to about 17% by weight of the coating dry weight. In at least one embodiment, the at least one plasticizer is dibutyl sebacate, and is present in an amount from about 12% to about 14% by weight of the coating dry weight. The relative proportions of the preferred polymer coat ingredients, notably the ratio of the at least one water-insoluble, water-permeable film-forming polymer: the at least one water-soluble polymer or substance : the at least one plasticizer, can be varied depending on the desired rate of release. The skilled artisan will appreciate that controlling the permeability and/or the amount of coating applied to the tablet cores can control the rate of release of the active. For example, the permeability of the preferred polymer coat can be altered by varying the ratio of the at least one water-insoluble, water-permeable film-forming polymer: the at least one water-soluble polymer : the at least one plasticizer and/or the quantity of coating applied to the tablet cores. A more delayed controlled-release is generally obtained with a higher amount of water-insoluble, water-permeable film forming polymer and/or a lower amount of the at least one water soluble polymer, and/or by increasing the amount of the coating solution applied to the tablet cores. Alternatively, a faster rate of release can be obtained by increasing the amount of the water-soluble polymer, decreasing the amount of the at least one water-insoluble water permeable film-forming polymer, and/or by decreasing the amount of coating solution applied. The addition of other excipients to the tablet core can also alter the permeability of the coat. For example, if it is desired that the tablet core further comprise an expanding agent, the amount of plasticizer in the coat can be increased to make the coat more pliable as the pressure exerted on a less pliable coat by the expanding agent can rupture the coat. Other excipients such as pigments and taste-masking agents can also be added to the coating formulation. In at least one embodiment, the proportions of the at least one water-insoluble water-permeable film forming polymer : the at least one water-soluble polymer : the at least one plasticizer for maintaining the integrity of the coat for at least about 24 hours and for obtaining the in vitro release profile described above is about 50-85 : 10-40 : 5-20. In at least one embodiment, the ratio is about 58-60 : 23-26 : 15-17. In at least one embodiment, the ratio is about 54-56 : 30-33 : 12-14.

The polymer coat is prepared and applied as follows. The appropriate amounts of the water-insoluble water-permeable film-forming polymer, preferably ethylcellulose, the water-soluble polymer, preferably polyvinylpyrrolidone, and plasticizer, preferably dibutyl sebacate are all dissolved in an alcoholic solvent such as ethanol, isopropyl alcohol, or a mixture thereof. The resulting coating solution is sprayed onto the tablet cores, using a coating pan apparatus. In at least one embodiment, the percentage weight gain resulting from application of the coating solution onto the cores ranges from about 2% to about 50% by weight of the uncoated cores. In at least one embodiment, the percentage weight gain resulting from application of the coating solution onto the cores ranges from about 8% to about 30% by weight of the uncoated cores. In at least one embodiment, the percentage weight gain resulting from application of the coating solution onto the cores ranges from about 10% to about 18% by weight of the uncoated cores. In at least one embodiment, the percentage weight gain resulting from application of the coating solution onto the cores ranges from about 12% to about 15% by weight of the uncoated cores. In at least one embodiment, the percentage weight gain resulting from application of the coating solution onto the cores ranges from about 15% to about 18% by weight of the uncoated cores.

Surprisingly, it was discovered that the above coating formulation provides for a delayed controlled-release composition even though the core has less than 10% of a gelling agent and the composition does not contain a pre-formed pore or passageway in the coating. Without wishing to be bound to any particular theory, it is believed that when a composition of the present invention is in the presence of an aqueous medium, the water-soluble polymer component of the release-controlling coating hydrates and swells. The hydrated coating at this point acts as a permeable membrane, and a flux of aqueous medium into the composition is established. This in turn dissolves the at least one form of venlafaxine from the core. The saturated solution of the at least one form of venlafaxine inside the coated tablet now establishes a flux gradient of the at least one form of venlafaxine going outwards through the permeable membrane to the aqueous medium. The flux on either side is controlled by the permeability of the membrane, which in turn depends on the amount of soluble polymer in the membrane. The rate of efflux of the at least one form of venlafaxine from inside the membrane is initially greater, gradually slowing down until sink conditions are established outside and inside the permeable membrane (following Fick's law of diffusion). Accordingly, the delayed release characteristics of the formulations of the invention do not depend upon enteric coatings or other pH-dependent release modifying coatings.

### Ethanol-induced Dose Dumping

In at least one embodiment, the enhanced absorption delayed controlled release composition of the present invention shows a reduced ethanol-induced dose dumping effect compared to the commercially available extended release formulation of venlafaxine, Effexor^{®} XR. The terms "dose dumping" or "ethanol-induced dose dumping"as used herein interchangeably are defined to mean the unintended premature release (*in-vitro*) of venlafaxine from a controlled release dosage form. The term "premature release" as used herein is defined to mean a release of venlafaxine from a controlled release dosage form in dissolution medium containing alcohol (for example, dissolution medium containing from about 5% to about 40% ethanol) wherein the rate of release is faster than the rate of release of venlafaxine from the identical controlled release dosage form in the otherwise identical dissolution medium not containing alcohol. For example, in the case of a controlled release dosage form which shows an ethanol-induced dose dumping effect, the rate of release of venlafaxine from the controlled release dosage form in the presence of ethanol can be greater than 2 times the rate of release of venlafaxine from the controlled release dosage form in the absence of ethanol.

The ethanol-induced dose dumping effect is measured by determining the dissolution profile of the composition in dissolution medium comprising up to about 40% (v/v) of Alcohol USP (e.g. 5% ethanol and 95% dissolution medium; 20% ethanol and 80% dissolution medium; or 40% ethanol and 60% dissolution medium) and comparing the measured dissolution profile to one measured in dissolution medium containing 0% ethanol. The terms "dissolution profile" or "release profile" as used herein interchangeably are defined to mean a quality control test conducted according to instructions found in the United States Pharmacopoeia (USP), i.e. using a USP apparatus design with a dissolution medium as found in the USP. Dissolution tests measure the rate and extent of dissolution of the active drug in an aqueous dissolution medium *in vitro.* The dissolution rate or in-vitro release rates of venlafaxine from the enhanced absorption delayed controlled release dosage forms of the present invention can be measured using one of many USP apparatus designs and dissolution media; non-limiting examples of which include a USP Type 1 apparatus design or USP Type 2 apparatus design, with an aqueous dissolution medium selected from water; deionized water; 0.1 N HCl; 0.1 N HCl with added sodium chloride (e.g. 15.7 g NaCl/Litre);0.1N HCl with added 0.1 % cetrimide; USP buffer pH 1.5; acetate buffer pH 4.5; phosphate buffer pH 6.5; phosphate buffer pH 6.8; and phosphate buffer pH 7.4. The terms "% released" and "% dissolved", when referring to a dissolution profile, are used interchangeably in this application and are defined to mean the percentage of the venlafaxine present in the dosage form which is released in an aqueous dissolution medium *(in vitro).*

The term "reduced ethanol-induced dose dumping effect" as used herein is defined to mean that the rate of release of venlafaxine from a controlled release dosage form in a dissolution medium containing from about 5% to about 40% ethanol, as determined by a dissolution profile measured as described herein, is less than about 2 times the rate of release of venlafaxine from an identical controlled release dosage form in a dissolution medium which contains 0% ethanol, as determined by a dissolution profile measured under otherwise identical conditions. For example, in certain embodiments, the rate of release of venlafaxine from a controlled release dosage form in dissolution media containing from about 5% to about 40% ethanol (for example, a dissolution medium containing from about 5% to about 40% ethanol and from about 60% to about 95% phosphate buffer pH 6.8) is less than about 2 times the rate of release of venlafaxine from the identical controlled release dosage form in dissolution media containing 0% alcohol (e.g. dissolution medium containing 100% phosphate buffer pH 6.8). In certain embodiments, the rate of release of venlafaxine from a controlled release dosage form in dissolution media containing from about 5% to about 40% ethanol (e.g. dissolution medium containing from about 5% to about 40% ethanol and from about 60% to about 95% phosphate buffer pH 6.8) is less than about 1.5 times the rate of release of venlafaxine from the identical controlled release dosage form in dissolution media not containing alcohol (e.g. dissolution medium containing about 100% phosphate buffer pH 6.8). In at least one embodiment, the rate of release of venlafaxine from the enhanced absorption delayed controlled release composition of the present invention in a dissolution medium containing from about 5% to about 40% ethanol is less than about 2 times the rate of release of venlafaxine from the enhanced absorption delayed controlled release composition in an otherwise identical dissolution medium which contains 0% ethanol. In at least one embodiment, the rate of release of venlafaxine from the enhanced absorption delayed controlled release composition of the present invention in a dissolution medium containing from about 5% to about 40% ethanol is less than about 1.5 times the rate of release of venlafaxine from the enhanced absorption delayed controlled release composition in an otherwise identical dissolution medium which contains 0% ethanol.

The following examples illustrate the present invention and are not intended to limit the scope of the present invention.

### EXAMPLE 1

### 30 mg Venlafaxine Delayed Controlled Release Tablets

The materials shown in Table 1 are combined to produce tablet cores for 30 mg venlafaxine delayed controlled release tablets:

**TABLE 1**

| Ingredients | Weight (mg) | % w/w |
|---|---|---|
| Venlafaxine Hydrochloride, USP | 33.95 | 24 |
| Polyvinyl Alcohol, USP¹ | 1.21 | 0.9 |
| Lactose #315 Spray Dried, USP² | 100.64 | 72 |
| Glyceryl Behenate, NF³ | 4.2 | 3 |
| Purified Water⁴, USP | N/A | N/A |
| Tablet Core Weight | 140 | 100 |

| | | |
|---|---|---|
| ¹Gelling Agent ²Filler ³Lubricant ⁴Evaporates after drying | | |

The venlafaxine hydrochloride and filler, Lactose 315 (Spray Dried), are first granulated with an aqueous solution of the gelling agent, polyvinyl alcohol, in a suitable fluid bed granulator apparatus. The granulate is subsequently dried and sieved through a 1.4 mm screen. The sized granules are next blended with more filler together with the lubricant, glyceryl behenate, in a V-blender and then compressed into tablets using a conventional rotary tablet press.

The dissolution of the resulting tablet cores is determined under the following conditions:
Medium: 1000 mL pH 6.8 phosphate buffer
Method: USP Type I Apparatus, 75 rpm at 37°C ±.0.5°C.

The data shows that greater than 90% of the venlafaxine hydrochloride is released in about 30 minutes.

The materials shown in Table 2 are combined to produce the modified release coat:

**TABLE 2**

| Ingredients | Weight (mg) | % w/w |
|---|---|---|
| Ethylcellulose 100, NF¹ | 12.6 | 60 |
| Povidone, USP² | 4.9 | 23.3 |
| Dibutyl Sebacate, NF³ | 3.5 | 16.6 |
| Ethyl Alcohol (200 proof), USP and Isopropyl Alcohol (99%), USP⁴ | N/A | N/A |
| Total Dry Solids (% weight gain) | 21 (15) | 100 |
| Tablet Cores | 140 | -- |
| Total Weight of Coated Tablet | 161 | -- |

| | | |
|---|---|---|
| ¹Water-insoluble water-permeable film forming polymer ²Water-soluble polymer ³Plasticizer ⁴Solvent, both evaporate after drying | | |

The plasticizer, dibutyl sebacate, is first dissolved in the solvent (ethyl alcohol / isopropyl alcohol mixture). The water-insoluble water-permeable film-forming polymer (ethylcellulose) is slowly added to the plasticizer/solvent mixture followed by the addition of the water-soluble polymer (Povidone) until a homogenous solution is achieved. Coating of the tablet cores from Example 1 is then carried out in an O'Hara Labcoat III System until an about 15% weight gain is achieved.

The tablets are coated until the desired weight gain is reached and subsequently dried at an inlet air temperature set at 50 ± 3°C, for 5 minutes at pan speed 2 rpm. Drying is continued for another 40 minutes at Jog with the same pan speed and the same parameters. The inlet temperature is subsequently turned off and the tablets cooled by keeping the exhaust on. The dissolution of the coated tablets is determined under the same experimental conditions as for the uncoated tablet cores. The results are presented in Table 3 as % released of the total venlafaxine hydrochloride in the coated tablet cores:

**TABLE 3**

| Time (hr) | % Released |
|---|---|
| 1 | 4 |
| 2 | 15 |
| 3 | 26 |
| 4 | 37 |
| 5 | 48 |
| 6 | 57 |
| 7 | 64 |
| 8 | 71 |
| 9 | 76 |
| 10 | 80 |
| 11 | 84 |
| 12 | 86 |
| 13 | 89 |
| 14 | 91 |
| 15 | 93 |
| 16 | 95 |
| 17 | 97 |
| 18 | 98 |
| 19 | 99 |
| 20 | 100 |
| 21 | 100 |
| 22 | 101 |
| 23 | 101 |
| 24 | 102 |

The release profile of the coated tablet cores compared to the release profile of the uncoated cores shows that the polymers if used in the granulation process to form the cores do not significantly impede the release of drug from the tablet. The polymer coat provides the delayed controlled release profile. This is also true for all dosage strengths of venlafaxine.

### EXAMPLE 2

### 60 mg Venlafaxine Delayed Controlled Release Tablets

The materials shown in Table 4 are combined to produce tablet cores for 60 mg venlafaxine delayed controlled release tablets:

**TABLE 4**

| Ingredients | Weight (mg) | % w/w |
|---|---|---|
| Venlafaxine Hydrochloride, USP | 67.90 | 42 |
| Polyvinyl Alcohol, USP¹ | 2.4 | 1.5 |
| Lactose #315 Spray Dried, USP² | 84.90 | 53 |
| Glyceryl Behenate, NF³ | 4.8 | 3 |
| Purified Water⁴, USP | N/A | N/A |
| Tablet Core Weight | 160 | 100 |

| | | |
|---|---|---|
| ¹Gelling Agent ²Filler ³Lubricant ⁴Evaporates after drying | | |

The tablet cores are manufactured as described in Example 1 and subsequently coated as also described in Example 1 with a solution of materials shown in Table 5:

**Table 5**

| Ingredients | Weight (mg) | % w/w |
|---|---|---|
| Ethylcellulose 100, NF¹ | 11.4 | 60 |
| Povidone, USP² | 4.43 | 23.3 |
| Dibutyl Sebacate, NF³ | 3.17 | 16.6 |
| Ethyl Alcohol (200 proof), USP and Isopropyl Alcohol (99%), USP⁴ | N/A | N/A |
| Total Dry Solids (% weight gain) | 19 (12) | 100 |
| Tablet Cores | 160 | -- |
| Total Weight of Coated Tablet | 179 | -- |

| | | |
|---|---|---|
| ¹Water-insoluble water-permeable film forming polymer ²Water-soluble polymer ³Plasticizer ⁴Solvent, both evaporate after drying | | |

The dissolution of the coated tablets is determined as described in Example 1 for the uncoated tablet cores. The results are presented in Table 6 as % released of the total venlafaxine hydrochloride coated tablet cores:

**TABLE 6**

| Time (hr) | % Released |
|---|---|
| 1 | 3 |
| 2 | 11 |
| 3 | 21 |
| 4 | 30 |
| 5 | 40 |
| 6 | 49 |
| 7 | 57 |
| 8 | 63 |
| 9 | 68 |
| 10 | 72 |
| 11 | 75 |
| 12 | 78 |
| 13 | 81 |
| 14 | 83 |
| 15 | 85 |
| 16 | 87 |
| 17 | 89 |
| 18 | 90 |
| 19 | 91 |
| 20 | 92 |
| 21 | 93 |
| 22 | 94 |
| 23 | 94 |
| 24 | 95 |

### EXAMPLE 3

### 120 mg Venlafaxine Delayed Controlled Release Tablets

The materials shown in Table 7 are combined to produce tablet cores for 120 mg venlafaxine delayed controlled release tablets:

**TABLE 7**

| Ingredients | Weight (mg) | % w/w |
|---|---|---|
| Venlafaxine Hydrochloride, USP | 135.80 | 42.4 |
| Polyvinyl Alcohol, USP¹ | 4.8 | 1.5 |
| Lactose #315 Spray Dried, USP² | 169.8 | 53 |
| Glyceryl Behenate, NF³ | 9.6 | 3 |
| Purified Water⁴, USP | N/A | N/A |
| Tablet Core Weight | 320 | 100 |

| | | |
|---|---|---|
| ¹Gelling Agent ²Filler ³Lubricant ⁴Evaporates after drying | | |

The tablet cores are manufactured and coated as described in Example 1 with a solution of materials shown in Table 8:

**TABLE 8**

| Ingredients | Weight (mg) | % w/w |
|---|---|---|
| Ethylcellulose 100, NF¹ | 27.53 | 58.58 |
| Povidone, USP² | 12.49 | 26.57 |
| Dibutyl Sebacate, NF³ | 6.98 | 14.8 |
| Ethyl Alcohol (200 proof), USP and Isopropyl Alcohol (99%), USP⁴ | N/A | N/A |
| Total Dry Solids (% weight gain) | 47 (15) | 100 |
| Tablet Cores | 320 | -- |
| Total Weight of Coated Tablet | 367 | -- |

| | | |
|---|---|---|
| ¹Water-insoluble water-permeable film forming polymer ²Water-soluble polymer ³Plasticizer ⁴Solvent, both evaporate after drying | | |

The dissolution of the coated tablets is determined as described in Example 1 for the uncoated tablet cores. The results are presented in Table 9 as % released of the total venlafaxine in the coated tablet cores:

**TABLE 9**

| Time (hr) | % Released |
|---|---|
| 1 | 4 |
| 2 | 11 |
| 3 | 21 |
| 4 | 31 |
| 5 | 43 |
| 6 | 53 |
| 7 | 63 |
| 8 | 70 |
| 9 | 77 |
| 10 | 82 |
| 11 | 86 |
| 12 | 90 |
| 13 | 92 |
| 14 | 94 |
| 15 | 96 |
| 16 | 97 |
| 17 | 98 |
| 18 | 98 |
| 19 | 99 |
| 20 | 100 |
| 21 | 100 |
| 22 | 100 |
| 23 | 100 |
| 24 | 95 |

### EXAMPLE 4

### 180 mg Venlafaxine Delayed Controlled Release Tablets

The materials shown in Table 10 are combined to produce tablet cores for 180 mg venlafaxine delayed controlled release tablets:

**TABLE 10**

| Ingredients | Weight (mg) | % w/w |
|---|---|---|
| Venlafaxine Hydrochloride, USP | 203.67 | 42.4 |
| Polyvinyl Alcohol, USP¹ | 7.2 | 1.5 |
| Lactose #315 Spray Dried, USP² | 254.73 | 53 |
| Glyceryl Behenate, NF³ | 14.4 | 3 |
| Purified Water⁴, USP | N/A | N/A |
| Tablet Core Weight | 480 | 100 |

| | | |
|---|---|---|
| ¹Gelling Agent ²Filler ³Lubricant ⁴Evaporates after drying | | |

The tablet cores are manufactured and subsequently coated as described in Example 1 with a coating solution of materials shown in Table 11:

**TABLE 11**

| Ingredients | Weight (mg) | % w/w |
|---|---|---|
| Ethylcellulose 100, NF¹ | 33.966 | 58.56 |
| Povidone, USP² | 15.39 | 26.53 |
| Dibutyl Sebacate, NF³ | 8.64 | 14.8 |
| Ethyl Alcohol (200 proof), USP and Isopropyl Alcohol (99%), USP⁴ | N/A | N/A |
| Total Dry Solids (% weight gain) | 58 (12) | 100 |
| Tablet Cores | 480 | -- |
| Total Weight of Coated Tablet | 538 | -- |

| | | |
|---|---|---|
| ¹Water-insoluble water-permeable film forming polymer ²Water-soluble polymer ³Plasticizer ⁴Solvent, both evaporate after drying | | |

The dissolution of the coated tablets is determined as described in Example 1 for the uncoated tablet cores. The results are presented in Table 12 as % released of the total venlafaxine hydrochloride in the coated tablet cores:

**TABLE 12**

| Time (hr) | % Released |
|---|---|
| 1 | 3 |
| 2 | 11 |
| 3 | 20 |
| 4 | 30 |
| 5 | 40 |
| 6 | 50 |
| 7 | 60 |
| 8 | 68 |
| 9 | 74 |
| 10 | 80 |
| 11 | 84 |
| 12 | 87 |
| 13 | 90 |
| 14 | 92 |
| 15 | 94 |
| 16 | 95 |
| 17 | 96 |
| 18 | 97 |
| 19 | 97 |
| 20 | 98 |
| 21 | 98 |
| 22 | 98 |
| 23 | 98 |
| 24 | 98 |

### EXAMPLE 5

### 120 mg Venlafaxine Delayed Controlled Release Tablets

The materials listed in Table 13 below are granulated in a suitable fluid bed granulator apparatus, such as a Glatt GPCG-60 air suspension processor.

**TABLE 13**

| *Granulating Substrate* | % w/w |
|---|---|
| Venlafaxine HCI | 45.52 |
| Lactose monohydrate (Lactose #315 monohydrate spray-dried) | 43.21 |
| Hydroxypropylmethylcellulose (Hypromellose, Methocel E3 Premium LV) | 5.37 |
| Colloidal Silicon dioxide (Aerosil 200) | 2.15 |
| | |
| *Granulating Solution* (*4.6% w*/*w):* | |
| Polyvinyl Alcohol (PVA) | 3.75 |
| Purified Water (evaporates on drying) | = |
| *Total* | 100.00 |

The granulate is subsequently dried and sieved through a 12 mesh screen. The sized granules are blended in a V-blender with lactose monohydrate and magnesium stearate in the amounts shown in Table 14 below, and compressed into tablet cores with an average mass of 320 mg, using a conventional rotary tablet press, such as a Kilian T200 tablet press.

**TABLE 14**

| *Tablet Ingredients:* | % w/w |
|---|---|
| Venlafaxine HCl Granules | 93.21 |
| Lactose monohydrate (Lactose #315 monohydrate spray-dried) | 6.29 |
| Magnesium Stearate NF, Non-Bovine | 0.50 |
| *Total* | 100.00 |

The dissolution of the resulting tablet cores is determined under the following conditions:
Medium: pH 6.8 phosphate buffer
Method: USP Type I Apparatus, 75 rpm at 37°C ±.0.5°C.

The results are presented in Table 15 as % released of the total venlafaxine hydrochloride in the uncoated tablet cores:

**TABLE 15**

| Time (minutes) | % Released (n=6) | | | |
|---|---|---|---|---|
| | Mean | Standard Deviation | Minimum | Maximum |
| 0 | 0 | 0 | 0 | 0 |
| 10 | 42 | 1 | 40 | 43 |
| 20 | 71 | 2 | 69 | 74 |
| 30 | 90 | 1 | 89 | 91 |
| 40 | 96 | 2 | 93 | 97 |
| 60 | 97 | 2 | 94 | 100 |
| 90 | 97 | 2 | 94 | 100 |
| 120 | 98 | 2 | 94 | 100 |

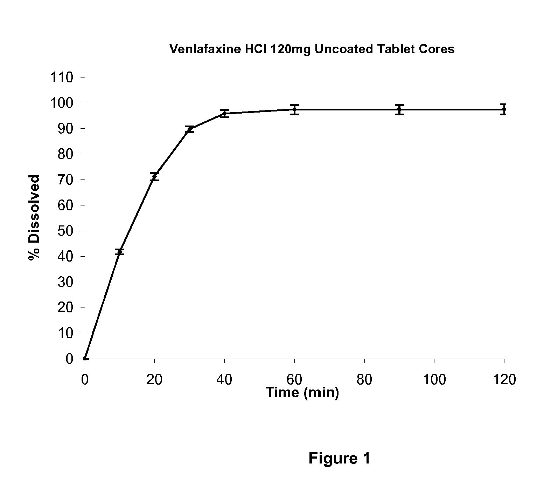

The data shows that at least about 90% of the venlafaxine hydrochloride is released in about 30 minutes. Figure 1 shows a chart of the data in Table 15.

The tablet cores are coated with the materials listed in Table 16 below.

**TABLE 16**

| *Coating Substrate:* | % w/w |
|---|---|
| Venlafaxine HCl 120 mg Uncoated Tablets | 85.47 |
| *Coating Solution (9*% *W*/*W): (excess solution may be prepared)* | |
| Ethylcellulose (Ethocel Standard 100 Premium) | 8.08 |
| Polyvinylpyrrolidone (Povidone; Kollidon 90F) | 4.52 |
| Dibutyl Sebacate | 1.93 |
| Ethyl Alcohol 190 Proof (evaporated during processing) | = |
| *Total* | 100.00 |

The coating solution is prepared by mixing the dibutyl sebacate and ethyl alcohol, then slowly adding the ethylcellulose followed by the polyvinylpyrrolidone, with stirring, until a homogenous solution is obtained. The tablet cores are coated with the coating solution at a temperature of 35 ± 10°C in a conventional vented coating pan, such as an O'Hara Labcoat I pan coater, until a coat weight gain corresponding to about 17% of the weight of the uncoated tablet cores is achieved. The coated tablets are subsequently dried for 3 minutes with the inlet heat on, then a further 10 minutes in jog mode with the inlet heat off. The final coated tablets have the composition listed in Table 17.

**TABLE 17**

| **Materials** | **Wt. (mg)** | **% w/w** |
|---|---|---|
| Venlafaxine HCI | 135.8 | 36.3 |
| Lactose 315 #1 | 149.0 | 39.8 |
| Methocel Pr E3LV | 16.0 | 4.3 |
| Aerosil 200 | 6.4 | 1.7 |
| Polyvinyl Alcohol | 11.2 | 3.0 |
| Magnesium Stearate | 1.6 | 0.4 |
| Ethocel 100 STD Premium | 29.9 | 8.0 |
| Kollidon 90F | 17.1 | 4.6 |
| Dibutyl sebacate | 7.4 | 2.0 |
| Ethyl alcohol 95% (evaporated during processing) | -- | -- |
| Total | 374.4 | 100.1 |

### EXAMPLE 6

### Effect of ethanol on the dissolution profile of 120 mg Venlafaxine Delayed Controlled Release Tablets

The dissolution profile of the 120 mg venlafaxine delayed controlled release tablets prepared as described in Example 5 is determined under the following conditions:

| | |
|---|---|
| Method: | USP Type I Apparatus, 75 rpm at 37°C ±.0.5°C. |
| Medium A: | pH 6.8 Phosphate buffer |
| Medium B: | 20% Ethanol / 80% pH 6.8 phosphate buffer |
| Medium C: | 40% Ethanol / 60% pH 6.8 phosphate buffer |

The results are presented in Tables 18 to 20 as % released of the total venlafaxine hydrochloride in the coated tablet cores. Figure 2 shows a chart of the data in Tables 18 to 20.

**TABLE 18**

| Time (hr) | % Released (Medium A) (n=5) | | | |
|---|---|---|---|---|
| | Mean | Minimum | Maximum | Standard. Deviation |
| 1 | 7 | 4 | 10 | 2 |
| 2 | 14 | 11 | 18 | 3 |
| 3 | 22 | 19 | 26 | 3 |
| 4 | 31 | 27 | 36 | 4 |
| 5 | 39 | 35 | 45 | 4 |
| 6 | 49 | 44 | 56 | 5 |
| 7 | 58 | 53 | 65 | 5 |
| 8 | 66 | 61 | 73 | 5 |
| 9 | 73 | 68 | 80 | 5 |
| 10 | 79 | 74 | 85 | 4 |
| 11 | 84 | 79 | 88 | 4 |
| 12 | 88 | 83 | 91 | 3 |
| 13 | 91 | 86 | 94 | 3 |
| 14 | 93 | 88 | 96 | 3 |
| 15 | 95 | 90 | 98 | 3 |
| 16 | 97 | 92 | 100 | 3 |
| 17 | 98 | 93 | 101 | 3 |
| 18 | 98 | 94 | 102 | 3 |
| 19 | 99 | 94 | 103 | 3 |
| 20 | 100 | 95 | 104 | 3 |

**TABLE 19**

| Time (hr) | % Released (Medium B) (n=6) | | | |
|---|---|---|---|---|
| | Mean | Minimum | Maximum | Standard. Deviation |
| 1 | 3 | 0 | 6 | 3 |
| 2 | 11 | 4 | 14 | 4 |
| 3 | 20 | 12 | 23 | 4 |
| 4 | 29 | 20 | 32 | 5 |
| 5 | 38 | 29 | 44 | 5 |
| 6 | 49 | 38 | 58 | 7 |
| 7 | 60 | 50 | 70 | 7 |
| 8 | 70 | 59 | 79 | 7 |
| 9 | 77 | 65 | 84 | 7 |
| 10 | 82 | 72 | 88 | 6 |
| 11 | 87 | 77 | 91 | 5 |
| 12 | 90 | 81 | 94 | 5 |
| 13 | 91 | 83 | 96 | 5 |
| 14 | 94 | 86 | 98 | 4 |
| 15 | 95 | 88 | 99 | 4 |
| 16 | 96 | 89 | 100 | 4 |
| 17 | 97 | 90 | 101 | 4 |
| 18 | 97 | 90 | 101 | 4 |
| 19 | 98 | 92 | 102 | 4 |

**TABLE 20**

| Time (hr) | % Released (Medium C) (n=6) | | | |
|---|---|---|---|---|
| | Mean | Minimum | Maximum | Standard. Deviation |
| 1 | 5 | 0 | 8 | 3 |
| 2 | 12 | 6 | 15 | 3 |
| 3 | 29 | 23 | 34 | 4 |
| 4 | 42 | 36 | 47 | 4 |
| 5 | 53 | 47 | 58 | 4 |
| 6 | 62 | 57 | 65 | 3 |
| 7 | 70 | 64 | 72 | 3 |
| 8 | 76 | 70 | 80 | 3 |
| 9 | 81 | 76 | 85 | 3 |
| 10 | 85 | 81 | 88 | 2 |
| 11 | 88 | 84 | 91 | 2 |
| 12 | 91 | 87 | 93 | 2 |
| 13 | 93 | 89 | 94 | 2 |
| 14 | 94 | 91 | 96 | 2 |
| 15 | 96 | 93 | 97 | 2 |
| 16 | 97 | 94 | 98 | 1 |
| 17 | 97 | 94 | 98 | 2 |
| 18 | 98 | 95 | 99 | 1 |
| 19 | 98 | 96 | 99 | 1 |
| 20 | 98 | 96 | 100 | 1 |

For the purposes of comparison, the dissolution profile of Effexor^{®} XR is determined under the following conditions:

| | |
|---|---|
| Method: | USP Type I Apparatus, 75 rpm at 37°C ±.0.5°C. |
| Medium D: | Deionized water |
| Medium E: | 40% Ethanol / 60% deionized water |

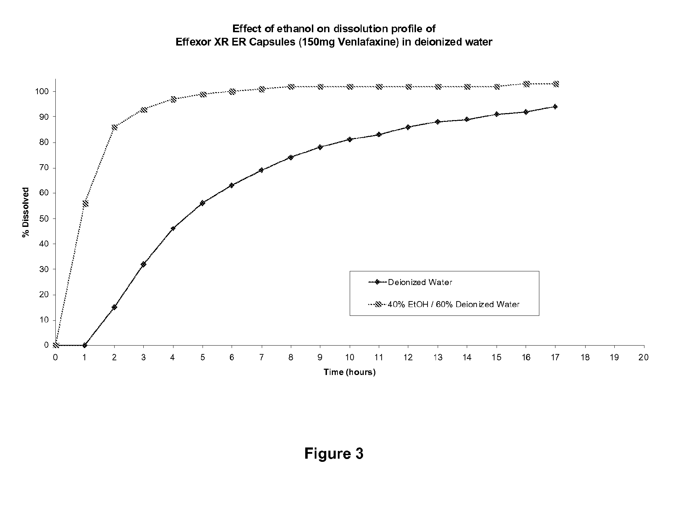

The results are presented in Tables 21 and 22 as % released of the total venlafaxine hydrochloride. Figure 3 shows a chart of the data in Tables 21 and 22.

**TABLE 21**

| Time (hr) | % Released (Medium D) (n=1) |
|---|---|
| 1 | 0 |
| 2 | 15 |
| 3 | 32 |
| 4 | 46 |
| 5 | 56 |
| 6 | 63 |
| 7 | 69 |
| 8 | 74 |
| 9 | 78 |
| 10 | 81 |
| 11 | 83 |
| 12 | 86 |
| 13 | 88 |
| 14 | 89 |
| 15 | 91 |
| 16 | 92 |
| 17 | 94 |

**TABLE 22**

| Time (hr) | % Released (Medium E) (n=1) |
|---|---|
| 1 | 56 |
| 2 | 86 |
| 3 | 93 |
| 4 | 97 |
| 5 | 99 |
| 6 | 100 |
| 7 | 101 |
| 8 | 102 |
| 9 | 102 |
| 10 | 102 |
| 11 | 102 |
| 12 | 102 |
| 13 | 102 |
| 14 | 102 |
| 15 | 102 |
| 16 | 103 |
| 17 | 103 |

The data shows that the 120 mg venlafaxine delayed controlled release formulation prepared according to Example 5 shows a reduced ethanol-induced dose dumping effect compared to the commercially available extended release formulation of venlafaxine, Effexor^{®} XR.

### EXAMPLE 7

### Pharmacokinetic Study Under Fed Conditions of 120 mg Venlafaxine Delayed Controlled Release Tablets

This randomized, open-label, single-dose, crossover, fed, Phase I (bioavailability) study is performed in compliance with Good Clinical Practice (GCP). The objective of this study is to determine and compare the rate and extent of absorption of venlafaxine and its metabolite, O-desmethylvenlafaxine, from a test formulation of Venlafaxine HCI Enhanced Absorption (EA) Extended Release (XR) 120 mg Tablets, prepared according to the procedure of Example 5, versus the reference Effexor XR^{®} (Venlafaxine HCI) 150 mg Extended-Release Capsules under fed conditions.

Following an overnight fast of at least 10 hours, and 30 minutes after the start of an American Heart Association (AHA) breakfast, either the test formulation (1 Venlafaxine HCI 120 mg EA XR Tablet, (potency value = 95.5% of label claim)) or the reference product (1 Effexor XR^{®} (Venlafaxine HCI) 150 mg Extended-Release Capsule, (potency value = 98.7% of label claim)) is administered orally with 240 mL of ambient temperature water. Study subjects are normal, healthy, non-smoking male and female subjects between the ages of 18 and 65 years. During each study period, 21 blood samples are collected from each subject at the following timepoints: 0.00 (pre-dose), 1.00, 2.00, 3.00, 4.00, 5.00, 6.00, 7.00, 8.00, 9.00, 10.00, 12.00, 14.00, 16.00, 18.00, 24.00, 30.00, 36.00, 48.00, 60.00, and 72.00 hours post-dose.

Pharmacokinetic and statistical analyses are performed on 14 subjects who completed the study. Venlafaxine and its active metabolite, O-desmethylvenlafaxine, in plasma are measured using a validated LC/MS/MS method. Pharmacokinetic parameters (AUC₀₋ₜ, AUC_{0-inf,}Cₘₐₓ, Tₘₐₓ, Kₑₗ, t_{½}, MRT, and M/P ratio) for venlafaxine and its metabolite O-desmethylvenlafaxine are calculated by standard non-compartmental methods. Using General Linear Model (GLM) procedures in Statistical Analysis System (SAS), analysis of variance (ANOVA) is performed on In-transformed AUC₀₋ₜ, AUC_{0-inf,}and Cₘₐₓ and on untransformed Kₑₗ, t_{½}, MRT, and M/P ratio at the significance level of 0.05. The intra-subject coefficient of variation (CV) is calculated using the Mean Square Error (MSE) from the ANOVA table. The ratio of geometric means and the 90% geometric confidence interval (90% C.I.) are calculated based on the difference in the Least Squares Means of the In-transformed AUC₀₋ₜ, AUC_{0-inf}, and Cₘₐₓ between the test and reference formulations. Tₘₐₓ is analyzed using nonparametric methods. The PAWC (Pharmacologic Activity-Weighted Composite) at each time point is calculated by multiplying the molar concentration of venlafaxine and O-desmethylvenlafaxine by their relative potencies and adding both concentrations. The relative potencies based on ED₅₀ for venlafaxine and O-desmethylvenlafaxine are 1:1.

Safety assessment is performed on all subjects who received at least 1 dose during the course of the study. The incidences of all adverse events (AEs) are tabulated by treatment and subject number. Absolute values for vital signs, electrocardiogram (ECG) parameters, laboratory parameters and physical examinations are also documented and values outside the normal range are flagged. Shifts from baseline values are tabulated. AEs are documented using investigator and Medical Dictionary for Regulatory Activities (MedDRA) terms.

**Pharmacokinetic Parameters for Venlafaxine:**

| **Pharmacokinetic Parameters** | **Geometric Mean (%CV) Arithmetic Mean ± SD** | |
|---|---|---|
| | **Venlafaxine HCl 120 mg EA XR Tablets (n=14)** | **Effexor XR^{®}(Venlafaxine HCl) Extended-Release Capsules 150 mg (n=14)** |
| | **Geometric Mean (%CV) Arithmetic Mean ± SD** | |
| **AUC**₀₋ₜ (ng·hr/mL) | 1003.34 (82.87) 1405.06 ± 1164.40 | 1056.71 (72.04) 1461.53 ± 1052.83 |
| **AUC**_{0-inf}(ng·hr/mL) | 1021.74 (83.08) 1429.22 ± 1187.42 | 1085.03 (72.59) 1496.67 ± 1086.40 |
| **C**ₘₐₓ (ng/mL) | 51.98 (70.61) 66.71 ± 47.10 | 56.26 (62.77) 69.56 ± 43.67 |
| Tₘₐₓ (hr) (median (min - max)) | 12.00 (5.00-14.00) | 6.50 (5.00-14.00) |
| t_{½} (hr) | 7.98 ± 3.84 | 10.19 ± 2.85 |
| **K**ₑₗ (hr⁻¹) | 9.98E-02 ± 3.28E-02 | 7.36E-02 ± 2.24E-02 |
| MRT (hr) | 18.35 ± 3.97 | 18.83 ± 4.38 |

The mean plasma concentration versus time curve for venlafaxine is shown in Figure 4.

**Relative Bioavailability Assessments for Venlafaxine:**

| **Parameter** | ***Potency Uncorrected Data*** | | | |
|---|---|---|---|---|
| | **90% C.I.** | **Ratio of Means** | | **Intra-Subject CV** |
| **AUC₀₋ₜ** | 85.05% to 106.01% | 94.95% | | 17.39% |
| **AUC_{0-inf}** | 84.39% to 105.08% | 94.17% | | 17.31% |
| **Cₘₐₓ** | 78.58% to 108.65% | 92.40% | | 25.80% |

| **Parameter** | ***Potency Corrected Data*** | | | |
|---|---|---|---|---|
| | **90% C.I.** | | **Ratio of Means** | |
| **AUC₀₋ₜ** | 87.90 % to 109.56 % | | 98.13 % | |
| **AUC_{0-inf}** | 87.22 % to 108.60 % | | 97.32 % | |
| **Cₘₐₓ** | 81.21% to 112.29% | | 95.49% | |

**Pharmacokinetic Parameters for O-Desmethylvenlafaxine:**

| **Pharmacokinetic Parameters** | **Geometric Mean (%CV) Arithmetic Mean ± SD** | |
|---|---|---|
| | **Venlafaxine HCl 120 mg EA XR Tablets (n=14)** | ^{®} **Effexor XR^{®} (Venlafaxine HCI) Extended-Release Capsules 150 mg (D) (n=14)** |
| **AUC**₀₋ₜ(ng·hr/mL) | 5337.20(23.13) 5484.61 ± 1268.68 | 5649.21(30.53) 5977.50 ± 1824.65 |
| **AUC**_{0-omf} (ng·hr/mL) | 5526.92 (24.72) 5687.57 ± 1406.00 | 5909.83 (31.32) 6258.16 ± 1960.22 |
| **C**ₘₐₓ (ng/mL) | 172.21 (27.28) 179.67 ± 49.02 | 179.48 (32_{.}71) 192.65 ± 63.01 |
| **T**ₘₐₓ (hr) (median (min - max)) | 16.00(12.00-24.00) | 11.00(8.00-14.00) |
| **t**_{½} (hr) | 11.73 ± 3.70 | 13.21 ± 2.98 |
| **K**ₑₗ (hr⁻¹) | 6.35E-02 ± 1.55E-02 | 5.52E-02 ± 1.33E-02 |
| **MRT** (hr) | 27.25 ± 5.40 | 27.55 ± 5.47 |
| **M/P Ratio** | 8.32 ± 7.51 | 8.81 ± 8.33 |

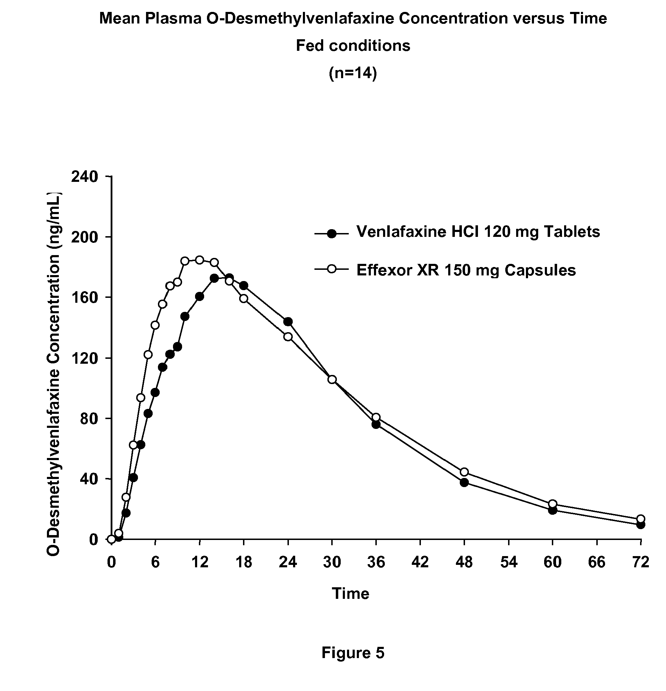

The mean plasma concentration versus time curve for O-desmethylvenlafaxine is shown in Figure 5.

**Relative Bioavailability Assessments for O-Desmethylvenlafaxine:**

| Parameter | ***Potency Uncorrected Data*** | | |
|---|---|---|---|
| | **90% C.I.** | **Ratio of Means** | **Intra-Subject CV** |
| **AUC**₀₋ₜ | 87.01% to 102.59% | 94.48% | 12.96% |
| **AUC**_{0-inf} | 85.89% to 101.83% | 93.52% | 13.40% |
| **C**ₘₐₓ | 86.44% to 106.51% | 95.95% | 16.47% |

**Pharmacokinetic Parameters for PAWC:**

| **Pharmacokinetic Parameters** | **Geometric Mean (%CV) Arithmetic Mean ± SD** | |
|---|---|---|
| | **Venlafaxine HCI 120 mg EA XR Tablets (n=14)** | **Effexor XR^{®}(Venlafaxine HCl) Extended-Release Capsules 150 mg (n=14)** |
| **AUC**₀₋ₜ (uM·hr) | 25.50 (18.78) 25.90±4.86 | 27.17 (25.67) 27.97±7.18 |
| **AUC**_{0-inf} (uM·hr) | 26.18(21.43) 26.70±5.72 | 28.21 (27.44) 29.14±8.00 |
| **Cₘₐₓ** (uM) | 0.88 (18.55) 0.89±0.17 | 0.93(21.78) 0.95±0.21 |
| **Tₘₐₓ** (hr) (median (min - max)) | 14.00(5.00-18.00) | 10.00(7.00-14.00) |
| **t**_{½} (hr) | 11.07±3.09 | 12.72±2.80 |
| **K**ₑₗ (hr⁻¹) | 6.60E-02 ± 1.37E-02 | 5.71 E-02 ± 1.31 E-02 |
| **MRT** (hr) | 25.36 ± 4.34 | 25.76 ± 4.61 |

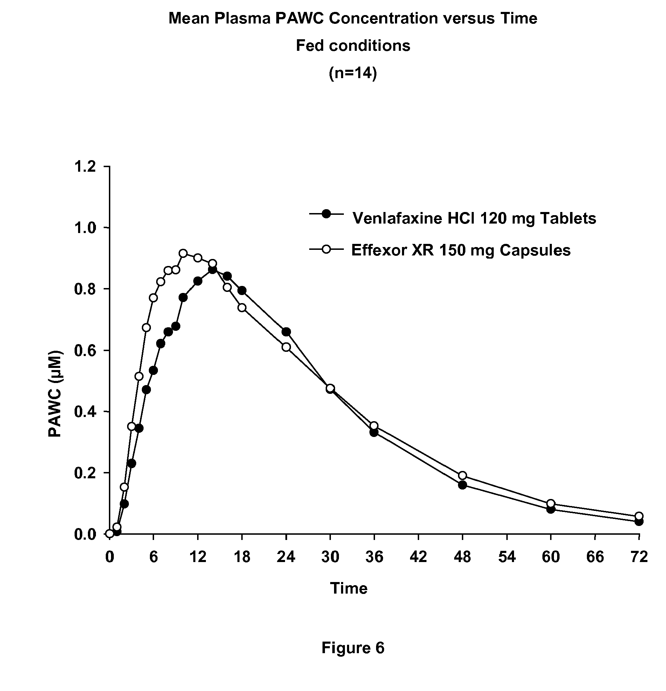

The mean plasma concentration versus time curve for the Pharmacologic Activity-Weighted Composite (PAWC) is shown in Figure 6.

**Relative Bioavailability Assessments for PAWC:**

| **Parameter** | ***Potency Uncorrected Data*** | | |
|---|---|---|---|
| | **90% C.I.** | **Ratio of Means** | **Intra-Subject CV** |
| **AUC**₀₋ₜ | 87.02% to 101.19% | 93.84% | 11.87% |
| **AUC**_{0-inf} | 85.95% to 100.26% | 92.83% | 12.11% |
| **C**ₘₐₓ | 85.48% to 105.33% | 94.88% | 16.47% |

For venlafaxine under fed conditions with a dose of 120 mg, the test product demonstrates an equivalent total exposure to a 150 mg dose of the reference product (Effexor XR^{®}) by exhibiting 90% Cl of the mean ratios of AUC values within 80.00 - 125.00% of that of the reference product. Similarly, the test formulation is bioequivalent to the reference product (Effexor XR^{®}) for the metabolite O-desmethylvenlafaxine (ODV) and the Pharmacologic Activity-Weighted Composite (PAWC). Overall, Venlafaxine HCI 120 mg EA XR Tablets are well tolerated as a single-dose of 120 mg, administered under fed conditions, and no significant safety issues emerged.

The results show that the venlafaxine delayed controlled release tablets formulated according to Example 5 administered at a dose of 120 mg of venlafaxine show bioequivalence to Effexor^{®} XR capsules administered at a dose of 150 mg of venlafaxine under fed conditions. Thus, the venlafaxine delayed controlled release tablets of the present invention have a higher bioavailability than Effexor^{®} XR and would be expected to show efficacy equivalent to the reference Effexor^{®} XR capsules while providing a lower dose of venlafaxine. Furthermore, the venlafaxine delayed controlled release tablets of the present invention would be expected to have a reduced or similar side effect or adverse event profile compared to the reference Effexor^{®} XR capsules.

### EXAMPLE 8

### Pharmacokinetic Study Under Fasted Conditions of 120 mg Venlafaxine Delayed Controlled Release Tablets

This randomized, open-label, single-dose, crossover, fasting, Phase I (bioavailability) study is performed in compliance with Good Clinical Practice (GCP). The objective of this study is to determine and compare the rate and extent of absorption of venlafaxine and its metabolite, O-desmethylvenlafaxine, from a test formulation of Venlafaxine HCI Enhanced Absorption (EA) Extended Release (XR) 120 mg Tablets, prepared according to the procedure of Example 5, versus the reference Effexor XR^{®} (Venlafaxine HCI) 150 mg Extended-Release Capsules under fasting conditions.

Following an overnight fast of at least 10 hours, either the test formulation (1 Venlafaxine HCI 120 mg EA XR Tablet, (potency value = 95.5% of label claim)) or the reference product (1 Effexor XR^{®} (Venlafaxine HCI) 150 mg Extended-Release Capsule, (potency value = 98.7% of label claim)) is administered orally with 240 mL of ambient temperature water. Study subjects are normal, healthy, non-smoking male and female subjects between the ages of 18 and 65 years. During each study period, 21 blood samples are collected from each subject at the following timepoints: 0.00 (pre-dose), 1.00, 2.00, 3.00, 4.00, 5.00, 6.00, 7.00, 8.00, 9.00, 10.00, 12.00, 14.00, 16.00, 18.00, 24.00, 30.00, 36.00, 48.00, 60.00, and 72.00 hours post-dose.

Pharmacokinetic and statistical analyses are performed on 10 subjects who completed the study. Venlafaxine and its active metabolite, O-desmethylvenlafaxine, in plasma are measured using a validated LC/MS/MS method. Pharmacokinetic parameters (AUC₀₋ₜ, AUC_{0-inf}, Cₘₐₓ, Tₘₐₓ, Kₑₗ, t_{½}, MRT, and M/P ratio) for venlafaxine and its metabolite O-desmethylvenlafaxine are calculated by standard non-compartmental methods. Using General Linear Model (GLM) procedures in Statistical Analysis System (SAS), analysis of variance (ANOVA) is performed on In-transformed AUC₀₋ₜ, AUC_{0-inf}, and Cₘₐₓ and on untransformed Kₑₗ, t_{½}, MRT, and M/P ratio at the significance level of 0.05. The intra-subject coefficient of variation (CV) is calculated using the Mean Square Error (MSE) from the ANOVA table. The ratio of geometric means and the 90% geometric confidence interval (90% C.I.) are calculated based on the difference in the Least Squares Means of the In-transformed AUC₀₋ₜ, AUC_{0-inf},and Cₘₐₓ between the test and reference formulations. Tₘₐₓ is analyzed using nonparametric methods. The PAWC (Pharmacologic Activity-Weighted Composite) at each time point is calculated by multiplying the molar concentration of venlafaxine and O-desmethylvenlafaxine by their relative potencies and adding both concentrations. The relative potencies based on ED₅₀ for venlafaxine and O-desmethylvenlafaxine are 1:1.

Safety assessment is performed on all subjects who received at least 1 dose during the course of the study. The incidences of all adverse events (AEs) are tabulated by treatment and subject number. Absolute values for vital signs, electrocardiogram (ECG) parameters, laboratory parameters and physical examinations are also documented and values outside the normal range are flagged. Shifts from baseline values are tabulated. AEs are documented using investigator and Medical Dictionary for Regulatory Activities (MedDRA) terms.

**Pharmacokinetic Parameters for Venlafaxine:**

| **Pharmacokinetic Parameters** | **Arithmetic Mean ± SD Geometric Mean (%CV)** | |
|---|---|---|
| | **Venlafaxine HCI 120 mg EA XR Tablets (n=10)** | **Effexor^{®} XR 150 mg Capsules (n=10)** |
| **AUC**₀₋ₜ (ng·hr/mL) | 1158.30 ± 828.38 947.52 (71.52) | 1223.21 ± 834.32 1020.49 (68.21) |
| **AUC**_{0-inf} (ng·hr/mL) | 1190.40±837.43 980.38(70.35) | 1271.46±864.05 1065.02 (67.96) |
| Cmax (ng/mL) | 80.52±46.95 71.59(58.31) | 88.76±44.82 79.86 (50.50) |
| **T**ₘₐₓ (hr) (median (min - max)) | 9.00(6.00-14.00) | 7.00(5.00-9.00) |
| t_{½} (hr) | 5.29 ± 1.36 | 9.85 ± 3.83 |
| **K**ₑₗ (hr⁻¹) | 1.37E-01 ± 2.86E-02 | 8.21 E-02 ± 3.66E-02 |
| **MRT** (hr) | 14.01 ± 2.56 | 16.15 ± 3.76 |

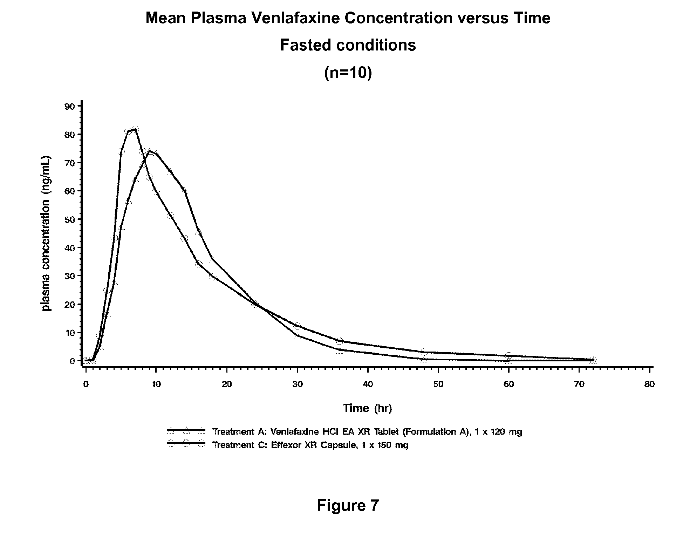

The mean plasma concentration versus time curve for venlafaxine is shown in Figure 7.

**Relative Bioavailability Assessments for Venlafaxine:**

| **Parameter** | ***Potency Uncorrected Data*** | | | |
|---|---|---|---|---|
| | **90% C.I.** | **Ratio of Means** | | **Intra-Subject CV** |
| **AUC**₀₋ₜ | 82.92% to 103.97% | 92.85% | | 13.67% |
| **AUC**_{0-inf} | 81.98% to 103.36% | 92.05% | | 14.00% |
| **C**ₘₐₓ | 72.25% to 111.21% | 89.64% | | 26.38% |

| **Parameter** | ***Potency Corrected Data*** | | | |
|---|---|---|---|---|
| | **90% C.I.** | | **Ratio of Means** | |
| **AUC**₀₋ₜ | 85.70% to 107.45% | | 95.96% | |
| **AUC**_{0-inf} | 84.73% to 106.82 % | | 95.14% | |
| **C**ₘₐₓ | 74.67% to 114.94 % | | 92.64% | |

**Pharmacokinetic Parameters for O-Desmethylvenlafaxine:**

| **Pharmacokinetic Parameters** | **Arithmetic Mean ± SD Geometric Mean (%CV)** | |
|---|---|---|
| | **Venlafaxine HCI 120 mg EA XR Tablets (n=10)** | **Effexor^{®} XR 150 mg Capsules (n=10)** |
| **AUC**₀₋ₜ (ng·hr/mL) | 4921.48 ± 1879.11 4610.04 (38.18) | 5851.09 ± 2348.25 5451.88 (40.13) |
| **AUC**_{0-inf} (ng·hr/mL) | 5022.01 ± 1887.51 4714.32 (37.58) | 6135.14 ± 2517.07 5698.43 (41.03) |
| Cmax (ng/mL) | 212.19 ± 70.38 202.69 (33.17) | 214.09 ± 56.33 207.94 (26.31) |
| **T**ₘₐₓ (hr) (median (min - max)) | 13.00 (8.00 - 16.00) | 10.00 (7.03 - 12.00) |
| t_{½} (hr) | 9.36 ± 1.45 | 12.75 ± 3.85 |
| **K**ₑₗ (hr⁻¹) | 7.57E-02 ± 1.18E-02 | 5.99E-02 ± 2.08E-02 |
| **MRT** (hr) | 21.66 ± 3.73 | 24.83 ± 5.80 |
| **M/P Ratio** | 5.98 ± 3.38 | 6.47 ± 3.46 |

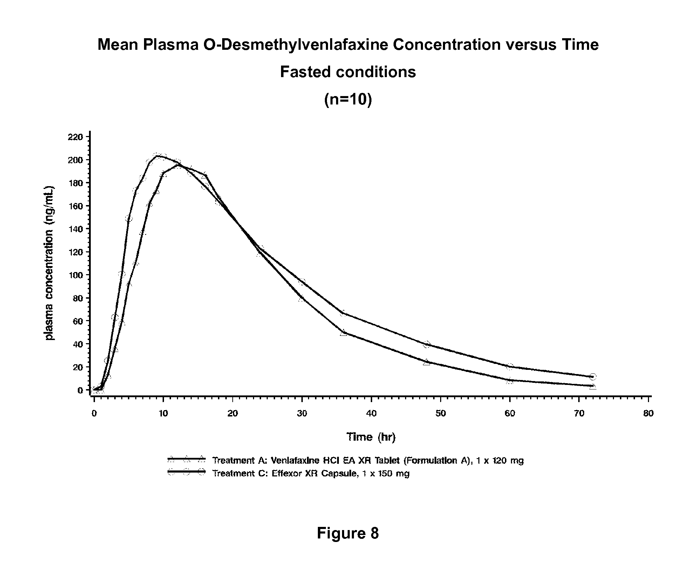

The mean plasma concentration versus time curve for O-desmethylvenlafaxine is shown in Figure 8.

**Relative Bioavailability Assessments for O-Desmethylvenlafaxine:**

| **Parameter** | **90% C.I.** | **Ratio of Means** | **Intra-Subject CV** |
|---|---|---|---|
| **AUC**₀₋ₜ | 76.96% to 92.90% | 84.56% | 11.36% |
| **AUC**_{0-inf} | 75.23% to 90.98% | 82.73% | 11.47% |
| **C**ₘₐₓ | 88.75% to 107.06% | 97.47% | 11.32% |

**Pharmacokinetic Parameters for PAWC:**

| **Pharmacokinetic Parameters** | **Arithmetic Mean ± SD Geometric Mean (%CV)** | |
|---|---|---|
| | **Venlafaxine HCI 120 mg EA XR Tablets (n=10)** | **Effexor^{®} XR 150 mg Capsules (n=10)** |
| **AUC**₀₋ₜ (µM·hr) | 22.93 ± 8.13 21.57 (35.47 | 26.68 ± 10.09 24.96 (37.81) |
| **AUC**_{0-inf} (µM-hr) **AUC**_{0-inf} (µM·hr) | 23.30 ± 8.16 21.95(35.01) | 27.79 ± 10.83 25.89 (38.97) |
| **C**ₘₐₓ (µM) **C**ₘₐₓ (µM) | 1.07 ± 0.32 1.03 (30.18) | 1.06 ± 0.24 1.04 (22.87) |
| **T**ₘₐₓ (hr) (median (min - max)) | 12.00 (8.00 - 14.00) | 8.00 (6.00 - 12.00) |
| **t**_{½} (hr) | 8.94 ± 1.45 | 12.39 ± 3.92 |
| **K**ₑₗ (hr⁻¹) | 7.94E-02 ± 1.27E-02 | 6.23E-02 ± 2.31 E-02 |
| **MRT** (hr) | 20.21 ± 3.49 | 23.29 ± 5.29 |

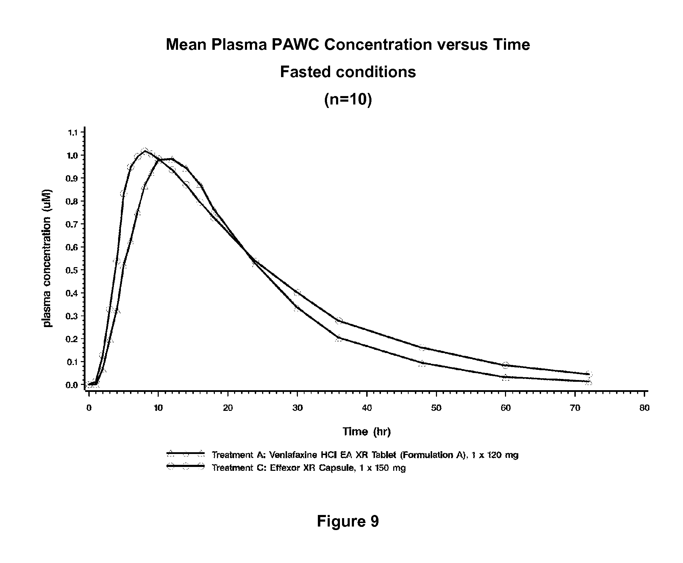

The mean plasma concentration versus time curve for the Pharmacologic Activity-Weighted Composite (PAWC) is shown in Figure 9.

**Relative Bioavailability Assessments for PAWC:**

| **Parameter** | **90% C.I.** | **Ratio of Means** | **Intra-Subject CV** |
|---|---|---|---|
| **AUC**₀₋ₜ | 79.24% to 94.26% | 86.42% | 10.47% |
| **AUC**_{0-inf} | 77.67% to 92.53% | 84.77% | 10.56% |
| **C**ₘₐₓ | 88.39% to 111.48% | 99.27% | 14.02% |

Overall, Venlafaxine HCI 120 mg EA XR Tablets were well tolerated as a single-dose of 120 mg, administered under fasting conditions, and no significant safety issues emerged.

## Claims

1. An enhanced absorption delayed controlled release pharmaceutical composition for oral administration suitable for once daily dosing comprising:
a) a core comprising by weight of the core dry weight from about 10% to about 90% of at least one form of venlafaxine, less than 10% of a gelling agent, and optional conventional excipients;
and
b) a modified release coat substantially surrounding the core, the coat comprising at least one water-insoluble, water-permeable, film-forming polymer, at least one water-soluble polymer or substance and at least one plasticizer;
wherein the composition provides a delayed controlled release of the at least one form of venlafaxine such that no more than 25% of the at least one form of venlafaxine is released after about 2 hours, about 15% to about 45% of the at least one form of venlafaxine is released after about 4 hours, about 50% to about 90% of the at least one form of venlafaxine is released after about 8 hours, no less than about 70% of the at least one form of venlafaxine is released after about 12 hours and no less than about 80% of the at least one form of venlafaxine is released after about 16 hours when tested using USP Apparatus 1 in 1000 ml of pH 6.8 phosphate buffer at 75 rpm at 37°C ± 0.5°C.

2. The enhanced absorption delayed controlled release composition of claim 1 wherein the composition shows a reduced ethanol-induced dose dumping effect compared to a reference composition, wherein the reference composition is a commercially available extended release formulation of venlafaxine.

3. The enhanced absorption delayed controlled release composition of claim 1 or 2 wherein the gelling agent comprises hydroxypropylmethylcellulose and polyvinyl alcohol.

4. The enhanced absorption delayed controlled release composition of any one of claims 1 to 3 wherein the at least one form of venlafaxine is venlafaxine hydrochloride.

5. The enhanced absorption delayed controlled release composition of any one of claims 1 to 4 which comprises from 20 to 200 mg of venlafaxine, preferably 120 mg of venlafaxine.

6. The enhanced absorption delayed controlled release composition of any one of claims 1 to 5 wherein the core comprises hydroxypropylmethylcellulose in an amount ranging from about 4% to about 6%, preferably about 5%, by weight of the core dry weight and polyvinyl alcohol in an amount ranging from about 1% to about 4%, preferably about 3.5%, by weight of the core dry weight.

7. The enhanced absorption delayed controlled release composition of any one of claims 1 to 6 wherein the at least one water-insoluble, water-permeable, film-forming polymer is ethylcellulose.

8. The enhanced absorption delayed controlled release composition of any one of claims 1 to 7 wherein the at least one water-insoluble, water-permeable, film-forming polymer is present in an amount ranging from about 20% to about 85% by weight of the coating dry weight.

9. The enhanced absorption delayed controlled release composition of any one of claims 1 to 8 wherein the at least one water-soluble polymer or substance is polyvinylpyrrolidone.

10. The enhanced absorption delayed controlled release composition of any one of claims 1 to 9 wherein the at least one water-soluble polymer or substance is present in an amount ranging from about 10% to about 75% by weight of the coating dry weight.

11. The enhanced absorption delayed controlled release composition of any one of claims 1 to 10 wherein the at least one plasticizer is dibutyl sebacate .

12. The enhanced absorption delayed controlled release composition of any one of claims 1 to 11 wherein the at least one plasticizer is present in an amount ranging from about 3% to about 40% by weight of the coating dry weight.

13. The enhanced absorption delayed controlled release composition of any one of claims 1 to 12 wherein the composition shows bioequivalence to a reference composition when the reference composition is administered at a dose which is at least about 1.20 times the dose at which the enhanced absorption delayed controlled release composition is administered, wherein the reference composition is a commercially available extended release formulation of venlafaxine.

14. The enhanced absorption delayed controlled release composition of any one of claims 1 to 13 wherein the rate of release of venlafaxine from the composition in a dissolution medium containing from about 5% to about 40% ethanol is less than about 2 times the rate of release of venlafaxine from the composition in an otherwise identical dissolution medium which contains 0% ethanol.

15. Use of an enhanced absorption delayed controlled release composition according to any one of claims 1 to 14 as an anti-depression agent.

16. Use of an enhanced absorption delayed controlled release composition according to any one of claims 1 to 14 in the preparation of a medicament for the treatment of depression.
